# FASCICULE DE BREVET EUROPEEN

(11) **EP 1 786 514 B1**
(45) Date de publication et mention de la délivrance du brevet: **07.01.2015**
(21) Numéro de dépôt: 05779697.1
(22) Date de dépôt: 24.06.2005
(51) Int. Cl.: A61N 1/32

(54) **DISPOSITIF OUR LE TRANSFERT DE MOLECULES AUX CELLULES UTILISANT UNE FORCE ELECTRIQUE**
VORRICHTUNG ZUM TRANSFER VON MOLEKÜLEN ZU ZELLEN UNTER VERWENDUNG VON ELEKTRISCHER ENERGIE
DEVICE FOR TRANSFERRING MOLECULES TO CELLS USING AN ELECTRIC FORCE

(30) Priorité: 24.06.2004 FR 0406943; 27.09.2004 FR 0410172; 20.01.2005 FR 0500603; 04.03.2005 FR 0502215
(43) Date de publication de la demande: 23.05.2007
(73) Titulaire: Sphergen, 94800 Villejuif (FR); SCHERMAN, Yves, F-94800 Villejuif (FR)
(72) Inventeur: SCHERMAN, Yves, F-94800 Villejuif (FR)
(74) Mandataire: Cabinet Plasseraud
(86) Numéro de dépôt international: PCT/FR2005/001611
(87) Numéro de publication internationale: WO 2006/010837

(56) Documents cités:
- WO-A-00/23143
- WO-A-99/04850
- WO-A-03/086534
- US-A1- 2003 097 089
- US-A1- 2004 092 860

## Description

### Domaine technique de l'invention

La présente invention concerne un dispositif permettant d'améliorer l'administration de substances dans des tissus in vivo et dans les cellules de ces tissus, en associant l'injection de principe actif à un processus physique.

Le processus physique utilisé est l'administration de champs électriques ou de courants électriques. Ces champs et/ou ce courant ont pour effet de perméabiliser les cellules de façon transitoire et aussi d'améliorer la pénétration du principe actif dans le tissu.

### Etat de la technique antérieure. Contexte de l'invention

L'électrotransfert ou l'électroperméabilisation consiste à injecter une molécule chimique ou un nucléotide dans un tissu, et à administrer simultanément ou après des impulsions de champs et de courant électrique, qui perméabilisent la paroi des cellules et par là même promeuvent l'entrée du nucléotide dans la cellule et, dans certains cas, jusqu'au noyau.

Ceci résulte d'un effet d'électrophorèse ou d'iontophorèse sur le principe actif, par lequel les molécules du principe actif sont entraînées par convection électriques dans les tissus ou au sein même des cellules humaines, animales, végétales ou bactériennes.

Dans la présente demande, nous appellerons "électrotransfert" ce procédé d'administration électriquement assistée, utilisant les champs électriques ou des courantes électriques pour améliorer l'administration d'un principe actif à des tissus biologiques, ainsi que son efficacité. Nous appellerons "champs" les champs électriques et électromagnétiques, ainsi que le courant électrique délivrés entre deux électrodes soumises à des tensions différentes.

La chimiothérapie antitumorale est un exemple dans lequel la pénétration de molécules chimiques à l'intérieur des cellules tumorales est nécessaire à une activité thérapeutique selon le procédé « électrochimiothérapie ».

L'électrotransfert d'ADN a pour effet de promouvoir la pénétration tissulaire et intracellulaire de ces ADN, ce qui rend possible leur expression sous forme d'ARN ou de protéine si ces ADN contiennent un gène précédé d'un promoteur et suivi d'une séquence de polyadénylation (ce que l'on appelle une cassette d'expression thérapeutique).

Dans la présente demande, on entend par "principe actif' toute molécule ayant un effet bénéfique ou à des fins d'analyse comme l'imagerie, fonctionnelle, et en particulier, toutes macromolécules de type peptide ou acide nucléique. Parmi les acides nucléiques, les plasmides ou les brins d'ADN ou d'ARN linéaires produits par synthèse sont une forme préférée. L'invention concerne aussi tout acide nucléique, toute protéine, tout sucre ou tout autre molécule biologique modifiée génétiquement ou chimiquement, ou encore toute molécule entièrement synthétique, fabriquée selon les procédés de l'homme de l'art.

L'électrotransfert peut être utilisé en particulier pour des ADN codant, des plasmides ou toute autre forme de matériel génétique (ADN, ARN ou autre) conduisant à l'expression du produit de ce gène, ce produit étant un ARN ou une protéine, notamment sur des tissus tel que muscles, tumeurs, peau, système nerveux ou foie.

L'électrotransfert nécessite un dispositif composé au minimum d'un générateur d'impulsions électriques et d'un dispositif d'électrodes.

Le brevet WO 99/373 58 décrit un appareil d'électroporation comprenant toutes les caractéristiques contenues dans le préambule de la revendication 1.

Le brevet US 5,273,525 décrit une seringue comportant deux aiguilles d'injection qui servent aussi d'électrodes.

Le brevet US n° 6,055,453 décrit l'utilisation d'un jeu d'électrodes identifiées permettant de sélectionner une séquence d'application de champs électriques.

Le brevet WO 03/086534 présente un système à deux électrodes aiguilles et une aiguille d'injection au centre.

Le brevet WO 02/098501 décrit un ensemble non ordonné d'électrodes aiguilles, l'une d'elle pouvant permettre d'injecter le principe actif à la surface de la peau.

Le brevet US 2004/0092860 propose en ensemble de plusieurs électrodes aiguilles servant d'injection, une aiguille d'injection se trouvant au centre.

Le brevet US5318514 décrit des dispositif d'électrodes.

Le brevet US98/08183 décrit des groupes configurations de réseaux d'électrodes.

Le brevet US2005070841 décrit un dispositif comportant deux aiguilles d'injection qui servent aussi d'électrodes

Le brevet US2005070841 décrit un dispositif comportant deux aiguilles d'injection qui servent aussi d'électrodes

Le brevet US 2003/0009148 décrit des électrodes en forme d'aiguilles invasive permettant d'intégrer en son centre une électrode tubulaire

Enfin, certains types de générateurs d'impulsion électriques sont déjà fabriqués et commercialisés.

### Description du problème

L'électrotransfert d'ADN implique la délivrance d'impulsions électriques sur une différence de tension pouvant être de plus de 30 V sur des durées pouvant être de l'ordre de grandeur de plusieurs dizaines de millisecondes. Ceci provoque des douleurs significatives, voir intolérables, et des contactions musculaires pouvant entraîner de grave séquelles parfois irréversibles. Or, la contraction musculaire peut avoir lieu même si le sujet est anesthésié. De plus, dans de nombreux cas, l'état de faiblesse ou l'âge du sujet rend l'anesthésie contre-indiquée.

### Solution apportée au problème

La présente invention propose des dispositifs d'électrodes invasives et non invasives et des méthodes permettant d'améliorer l'efficacité et l'innocuité de l'électrotransfert et de diminuer sa toxicité et l'importance des contractions musculaires.

La présente invention permet aussi une amélioration de l'efficacité en optimisant la position des électrodes et le choix des champs délivrés. Par "délivrer" ou "délivrance" des champs, nous entendons par la présente demande la génération par le générateur d'une différence de potentiel entre les électrodes, générant ainsi un courant électrique et/ou des champs électriques entre les électrodes. L'amélioration de l'efficacité donne une marge permettant si nécessaire d'améliorer l'innocuité en diminuant les paramètres engendrant douleur et contraction musculaire. La présente invention propose un dispositif de boiter permettant la prise en main du dispositif d'électrodes et de relier électriquement ces dernières au générateur.

Dans la présente demande, par "un jeu d'électrodes", on entend un ensemble d'au moins 1 électrode.

La présente invention propose les dispositifs et méthodes qui suivent.

La présente invention est composée d'un dispositif pour améliorer la pénétration in vivo de molécules de principe actif dans les cellules des tissus d'un sujet humain ou animal, caractérisé en ce qu'il comprend :
- un générateur d'impulsions électriques
- un premier ensemble d'électrodes composé d'au moins une électrode reliée électriquement à une première borne du générateur d'impulsions électriques,
- un deuxième ensemble d'électrodes composé d'au moins une électrode reliée électriquement à la seconde borne du générateur d'impulsions électriques, et
- un moyen d'injecter le principe actif dans les tissus.

En complément de la caractéristique décrite ci-dessus, une caractéristique possible de l'invention est que premier ensemble d'électrodes est composé d'une seule électrode.

En complément de certaines des caractéristiques décrites ci-dessus, une caractéristique possible de l'invention est que l'électrode est invasive.

En complément de certaines des caractéristiques décrites ci-dessus, une caractéristique possible de l'invention est que l'électrode dispose d'un moyen d'injecter le principe actif.

En complément de certaines des caractéristiques décrites ci-dessus, une caractéristique possible de l'invention est que l'électrode est une aiguille d'injection

En complément de certaines des caractéristiques décrites ci-dessus, une caractéristique possible de l'invention est que un moyen d'injection du principe actif est constitué d'au moins une aiguille solidaire et placée à proximité de l'électrode et de profondeur inférieure à la profondeur de l'électrode

En complément de certaines des caractéristiques décrites ci-dessus, une caractéristique possible de l'invention est que:
- L'électrode est constituée en outre d'une enveloppe cathéter percée d'orifices de taille suffisante pour permettre un contact entre l'aiguille et les tissus au travers de ces orifices.
- L'aiguille invasive de l'électrode traverse le cathéter par son axe et peut coulisser le long de l'axe du cathéter et pouvant être retirée du cathéter
- Le cathéter et l'aiguille, une fois assemblés, forment une électrode invasive ces électrodes étant ci-après appelées "électrode recouverte partiellement d'un Cathéter non conducteur"

En complément de certaines des caractéristiques décrites ci-dessus, une caractéristique possible de l'invention est que l'électrode est constituée par:
- un cathéter, recouvert d'une surface conductrice électriquement reliée à la borne correspondante du générateur d'impulsion, ci après nommé "électrode cathéter conductrice", sa surface étant composée d'un matériaux conservant approximativement la souplesse du cathéter, ce cathéter pouvant servir de moyen d'injection du principe actif
- une aiguille invasive traversant le cathéter par son axe et permettant de pénétrer les tissus et pouvant coulisser le long de l'axe du cathéter et pouvant être retirée du cathéter, l'aiguille pouvant aussi servir de moyen d'injection

En complément de certaines des caractéristiques décrites ci-dessus, une caractéristique possible de l'invention est que l'électrode invasive est recouverte dans sa partie supérieure pénétrant dans les tissus, d'un isolant électrique.

En complément de certaines des caractéristiques décrites ci-dessus, une caractéristique possible de l'invention est qu'au moins une électrode cathéter est aimantée
En complément de certaines des caractéristiques décrites ci-dessus, une caractéristique possible de l'invention est que le deuxième ensemble d'électrodes comporte au moins une électrode non-invasive disposés à la surface des tissus recouvrant la zone contenant le principe actif.

En complément de certaines des caractéristiques décrites ci-dessus, une caractéristique possible de l'invention est qu'au moins une électrode non invasive dispose d'un orifice permettant à l'électrode invasive de la traverser

En complément de certaines des caractéristiques décrites ci-dessus, une caractéristique possible de l'invention est qu'elle comprend en outre un guide permettant de diriger l'axe de l'électrode invasive selon une direction prédéfinie

En complément de certaines des caractéristiques décrites ci-dessus, une caractéristique possible de l'invention est que le deuxième ensemble d'électrode comporte une seule électrode invasive.

En complément de certaines des caractéristiques décrites ci-dessus, une caractéristique possible de l'invention est que l'électrode du deuxième ensemble est une électrode recouverte partiellement d'un Cathéter non conducteur.

En complément de certaines des caractéristiques décrites ci-dessus, une caractéristique possible de l'invention est que le l'électrode du deuxième ensemble est constituée d'une électrodes Cathéter conductrice.

En complément de certaines des caractéristiques décrites ci-dessus, une caractéristique possible de l'invention est que l'électrode du premier ensemble est une électrode Cathéter. et où les deux aiguilles sont parallèles, solidaires en sont reliées par un support.

En complément de certaines des caractéristiques décrites ci-dessus, une caractéristique possible de l'invention est que les deux électrodes invasives sont solidaires, assemblées à l'aide d'un support, approximativement parallèles et approximativement de même profondeur

En complément de certaines des caractéristiques décrites ci-dessus, une caractéristique possible de l'invention est que le deuxième ensemble d'électrode comporte une pluralité d'électrodes invasives.

En complément de certaines des caractéristiques décrites ci-dessus, une caractéristique possible de l'invention est que les électrodes invasives du deuxième ensemble sont situées approximativement sur un cercle dont l'électrode du premier ensemble forme approximativement au centre.

En complément de certaines des caractéristiques décrites ci-dessus, une caractéristique possible de l'invention est que les électrodes invasives du deuxième ensemble bordent la zone où est injecté le principe actif.

En complément de certaines des caractéristiques décrites ci-dessus, une caractéristique possible de l'invention est que deux électrodes invasives du deuxième ensemble d'électrodes sont sensiblement alignées avec l'électrode du premier ensemble.

En complément de certaines des caractéristiques décrites ci-dessus, une caractéristique possible de l'invention est qu'il comprend outre un moyen permettant d'orienter les électrodes selon un axe parallèle

En complément de certaines des caractéristiques décrites ci-dessus, une caractéristique possible de l'invention est que toutes les électrodes sont solidaires, assemblées à l'aide d'un support, approximativement parallèles et approximativement de même profondeur

En complément de certaines des caractéristiques décrites ci-dessus, une caractéristique possible de l'invention est que le dispositif comprenne en outre au moins une électrode non-invasive reliée à l'une des bornes du générateur

En complément de certaines des caractéristiques décrites ci-dessus, une caractéristique possible de l'invention est que:
- Chaque électrode invasive peut disposer d'un boîtier permettant une bonne prise en main de l'électrode et assurant la liaison électrique entre l'électrodes et sa borne du générateur.
- le boîtier disposant d'un habitacle permettant de loger l'électrodes et le moyen d'injection du principe actif et le réservoir contenant le principe actif

En complément de certaines des caractéristiques décrites ci-dessus, une caractéristique possible de l'invention est que:
- une pluralité d'électrodes dispose du même boîtier permettant une bonne prise en main du dispositif d'électrode et assurant la liaison électrique entre chaque électrode et sa borne du générateur respective.
- le boîtier dispose d'un habitacle permettant de loger les électrodes et le moyen d'injection du principe actif et le réservoir contenant le principe actif

En complément de certaines des caractéristiques décrites ci-dessus, une caractéristique possible de l'invention est que le boîtier comprend en outre le moyen d'orienter les électrodes selon un axe parallèle

En complément de certaines des caractéristiques décrites ci-dessus, une caractéristique possible de l'invention est que:
- les électrodes solidaires et leur support disposent d'un boîtier permettant une bonne prise en main du dispositif d'électrode et assurant la liaison électrique entre chaque électrode et sa borne du générateur.
- le boîtier dispose d'un habitacle permettant de loger les électrodes solidaires et leur support et le moyen d'injection du principe actif et le réservoir contenant le principe actif

En complément de certaines des caractéristiques décrites ci-dessus, une caractéristique possible de l'invention est que le boîtier :
- dispose d'un moyen permettant d'enfoncer successivement l'électrode dans les tissus à des profondeurs intermédiaires prédéfinies,
- et dispose d'un moyen permettant d'injecter le principe actif à chaque position d'arrêt.

En complément de certaines des caractéristiques décrites ci-dessus, une caractéristique possible de l'invention est que le moyen permettant d'enfoncer successivement les électrodes dans les tissus à des profondeurs intermédiaires prédéfinies est constitué d'au moins une butée.

En complément de certaines des caractéristiques décrites ci-dessus, une caractéristique possible de l'invention est qu'une pluralité d'électrodes invasives reliées au générateur sont recouvertes dans leur partie supérieure pénétrant dans les tissus, d'un isolant électrique.

En complément de certaines des caractéristiques décrites ci-dessus, une caractéristique possible de l'invention est que toutes les électrodes invasives reliées au générateur sont recouvertes dans leur partie supérieure pénétrant dans les tissus, d'un isolant électrique.

En complément de certaines des caractéristiques décrites ci-dessus, une caractéristique possible de l'invention est que les deux ensembles d'électrodes comportent un jeu d'électrodes non-invasives disposées à la surface des tissus recouvrant la zone contenant le principe actif.

En complément de certaines des caractéristiques décrites ci-dessus, une caractéristique possible de l'invention est que les électrodes sont appliquées sur une même face des tissus contenant le principe actif de façon à permettre la délivrance de champs électriques se propageant sous la surface des tissus sur lesquels les électrodes sont appliquées.

En complément de certaines des caractéristiques décrites ci-dessus, une caractéristique possible de l'invention est que la surface en contact avec les tissus d'au moins une électrode non-invasive a une forme approximativement rectangulaire

En complément de certaines des caractéristiques décrites ci-dessus, une caractéristique possible de l'invention est que la surface en contact avec les tissus d'au moins une électrode non-invasive a une forme ressemblant approximativement à un fer à cheval

En complément de certaines des caractéristiques décrites ci-dessus, une caractéristique possible de l'invention est que la surface d'au moins une électrode non-invasive est souple

En complément de certaines des caractéristiques décrites ci-dessus, une caractéristique possible de l'invention est qu'une électrode non-invasive a la forme d'une pointe aplatie à son extrémité

En complément de certaines des caractéristiques décrites ci-dessus, une caractéristique possible de l'invention est que les électrodes non-invasives font partie intégrante d'une gaine élastique.

En complément de certaines des caractéristiques décrites ci-dessus, une caractéristique possible de l'invention est qu'au moins une électrode non-invasive a une forme de fils.

En complément de certaines des caractéristiques décrites ci-dessus, une caractéristique possible de l'invention est que le dispositif ne comporte que 2 électrodes non-invasives en forme de fils, ces électrodes suivant un axe différent.

En complément de certaines des caractéristiques décrites ci-dessus, une caractéristique possible de l'invention est que la surface en contact avec les tissus d'au moins une électrode non-invasive a une forme irrégulière

En complément de certaines des caractéristiques décrites ci-dessus, une caractéristique possible de l'invention est que les électrodes non-invasives sont asymétriques.

En complément de certaines des caractéristiques décrites ci-dessus, une caractéristique possible de l'invention est que d'au moins une électrode non invasive est composée de plusieurs électrodes non invasive reliées électriquement entre elles

Le procédé suivant met en oeuvre un dispositif dont les caractéristiques possibles sont décrites ci dessus. Ce procédé permet d'améliorer la pénétration de molécules de principe actif in vivo dans les cellules des tissus d'un sujet humain ou animal; ce procédé comprenant les étapes suivantes:
- on met en contact avec les tissus à traiter au moins une électrode reliée électriquement à la première borne d'un générateur d'impulsion et au moins une électrode reliées électriquement à la deuxième borne du générateur d'impulsion et on injecte le principe actif dans la zone de tissus à traiter;
- puis on déclenche l'émission d'impulsions électriques par le générateur, l'amplitude des signaux électriques étant calculée en fonction de la distance entre les électrodes et de la nature des tissus, de façon à créer un champ électrique entre les électrodes, ceci permettant la pénétration du principe actif dans les tissus et dans les cellules;

En complément de la caractéristique décrite ci-dessus, une caractéristique possible du procédé est que le principe actif est injecté dans une cavité fermée qui contient les tissus à traiter et contenant une matière fluide.

En complément de certaines des caractéristiques décrites ci-dessus, une caractéristique possible du procédé est que le principe actif est injecté dans la cavité synoviale.

En complément de certaines des caractéristiques décrites ci-dessus, une caractéristique possible du procédé est que, avant de délivrer les champs:
- on enfonce successivement dans les tissus un jeu d'électrodes invasives reliées aux deux bornes du générateur à des profondeurs intermédiaires
- on injecte dans les tissus le principe actif à des profondeurs successives à l'aide desdites électrodes,
- puis on enfonce à une profondeur finale prédéfinie toutes les électrodes invasives avant de déclencher la délivrance des champs électriques.

En complément de certaines des caractéristiques décrites ci-dessus, une caractéristique possible du procédé est que, avant de délivrer les champs:
- les électrodes invasives reliées à la première borne du générateur sont introduites à la même profondeur dans les tissus selon le même axe et au centre de la zone contenant le principe actif
- les électrodes invasives reliées à la deuxième borne du générateur sont introduites dans les tissus, dans le même axes et sensiblement à une même profondeur que les électrodes invasives reliées à la première borne du générateur, en bordant approximativement la zone de tissus contenant le principe actif, les électrodes étant positionnées à distance approximativement identique du centre de la zone contenant le principe actif et étant réparties régulièrement autour de ce centre.

En complément de certaines des caractéristiques décrites ci-dessus, une caractéristique possible du procédé est qu'on injecte le principe actif à l'aide des électrodes reliées à la première borne du générateur

En complément de certaines des caractéristiques décrites ci-dessus, une caractéristique possible du procédé est que, avant de délivrer les champs:
- on relie au moins une électrode Cathéter à une borne d'un générateur
- on relie au moins électrode non-invasive à l'autre borne du générateur
- on enfonce chaque électrode Cathéter dans les tissus afin de pénétrer dans la cavité; on fait ensuite coulisser l'aiguille à l'intérieur de chaque cathéter de façon à ne pas endommager les parois de la cavité lors de déplacement de l'électrode Cathéter à l'intérieur de la cavité et lors de la délivrance des champs.
- on injecte le principe actif à l'aide d'au moins une électrode Cathéter
- on pose chaque électrode non-invasives au bord de la cavité

En complément de certaines des caractéristiques décrites ci-dessus, une caractéristique possible du procédé est qu'au moins une électrode Cathéter est reliée à une borne d'un générateur, au moins une électrode cathéter est reliée à l'autre borne du générateur et, avant de délivrer les champs:
- on enfonce chaque électrodes Cathéter dans les tissus afin de pénétrer dans la cavité ; on fait ensuite coulisser l'aiguille de chaque cathéter de façon à ne pas endommager les parois de la cavité lors de déplacement de l'électrode Cathéter à l'intérieur de la cavité et lors de la délivrance des champs.
- les électrodes Cathéter étant enfoncées de façon à être approximativement parallèles
- on injecte principe actif à l'aide d'au moins une électrode Cathéter

En complément de certaines des caractéristiques décrites ci-dessus, une caractéristique possible du procédé est qu'au moins une électrode non-invasive est en outre reliée aux bornes du générateur et posée sur les tissus bordant de la cavité

En complément de certaines des caractéristiques décrites ci-dessus, une caractéristique possible du procédé est que l'électrode non-invasive est pressée contre les tissus afin de se rapprocher de chaque électrode cathéter

En complément de certaines des caractéristiques décrites ci-dessus, une caractéristique possible du procédé est que l'une au moins des électrodes est une électrode cathéter conductrice utilisée pour injecter le principe actif en ayant préalablement enlevé l'aiguille du cathéter

En complément de certaines des caractéristiques décrites ci-dessus, une caractéristique possible du procédé est que ce qu'au moins une aiguille d'une électrode-Cathéter est utilisée pour injecter le principe actif

En complément de certaines des caractéristiques décrites ci-dessus, une caractéristique possible du procédé est que l'une au moins des électrodes est une électrode cathéter conductrice et en ce que l'aiguille est retirée totalement de son cathéter respectif avant la délivrance des champs

En complément de certaines des caractéristiques décrites ci-dessus, une caractéristique possible du procédé est que l'on injecte de manière continue le principe actif au fur et à mesure que l'on enfonce les électrodes

En complément de certaines des caractéristiques décrites ci-dessus, une caractéristique possible du procédé est que l'on injecte par étapes successives le principe actif au fur et à mesure que l'on enfonce les électrodes

En complément de certaines des caractéristiques décrites ci-dessus, une caractéristique possible du procédé est qu'au moins une électrode invasive est prise en main à l'aide d'un boîtier permettant aussi de la relier électriquement à sa borne du générateur et permettant une bonne prise en mains de l'électrode.

En complément de certaines des caractéristiques décrites ci-dessus, une caractéristique possible du procédé est que, une fois que les électrodes Cathéter sont introduites dans la cavité on utilise un moyen pour modifier l'angle de l'axe des électrodes afin d'obtenir un bon parallélisme et d'éviter tout risque de contact entre des électrodes rattachées à des bornes différentes du générateur.

En complément de certaines des caractéristiques décrites ci-dessus, une caractéristique possible du procédé est que, une fois que les électrodes Cathéter sont introduites dans la cavité, on applique un dispositif physique sur l'extrémité des électrodes ne pénétrant pas dans les tissus afin de modifier leur angle respectif et de les rendre parallèle

En complément de certaines des caractéristiques décrites ci-dessus, une caractéristique possible du procédé est qu'on utilise un moyen de connaître la distance entre les extrémités des électrodes, et adapte la position relative des électrodes afin d'obtenir le champ électrique ou le courant électrique souhaités en fonction des tissus ciblés.

En complément de certaines des caractéristiques décrites ci-dessus, une caractéristique possible du procédé est qu'on utilise un moyen de connaître la distance entre les extrémités des électrodes, et on adapte la programmation du générateur afin d'appliquer la tension permettant d'obtenir le champ électrique ou le courant électrique souhaité en fonction des tissus ciblés.

En complément de certaines des caractéristiques décrites ci-dessus, une caractéristique possible du procédé est que le générateur dispose d'un moyen de connaître en permanence la distance entre les extrémités des électrodes , et peut modifier dynamiquement la programmation des impulsions afin d'appliquer les paramètres permettant d'obtenir le champ électrique ou le courant électrique souhaités en fonction des tissus ciblés.

En complément de certaines des caractéristiques décrites ci-dessus, une caractéristique possible du procédé est que les électrodes invasives sont parallèles, rendues solidaires et maintenues à l'aide d'un boîtier permettant une bonne prise en mains du dispositif et permettant de relier les électrodes à leur borne de générateur respective

En complément de certaines des caractéristiques décrites ci-dessus, une caractéristique possible du procédé est que le premier ensemble d'électrode est composé d'une électrode invasive, et qu'au moins une électrode du deuxième ensemble d'électrodes est non-invasive et est positionnée à la surface des tissus contenant le principe

En complément de certaines des caractéristiques décrites ci-dessus, une caractéristique possible du procédé est qu'on enfonce au moins une électrode invasives du premier ensemble d'électrodes au travers des organes afin d'atteindre la zone de tissus contenant le principe actif sans traverser l'électrode non-invasive

En complément de certaines des caractéristiques décrites ci-dessus, une caractéristique possible du procédé est que les électrodes invasives sont enfoncées selon un axe faisant partie d'un plan sensiblement parallèle au plan occupé par la surface de la pluralité d'électrode non-invasive en contact avec la zone de tissus contenant le principe actif.

En complément de certaines des caractéristiques décrites ci-dessus, une caractéristique possible du procédé est qu'on isole électriquement la partie supérieure d'au moins une électrode invasive afin d'éviter le passage de courant électrique parasite dans le volume de tissus situé d'une part entre les électrodes et située d'autre part entre la surface de la peau et la zone de tissus contenant le principe actif.

En complément de certaines des caractéristiques décrites ci-dessus, une caractéristique possible du procédé est qu'on isole électriquement la partie supérieure de toutes électrodes invasives afin d'éviter le passage de courant électrique parasite dans le volume de tissus situé d'une part entre les électrodes et située d'autre part entre la surface de la peau et la zone de tissus contenant le principe actif.

En complément de certaines des caractéristiques décrites ci-dessus, une caractéristique possible du procédé est que l'on dispose des électrodes non-invasive du premier ensemble d'électrodes et du deuxième ensemble d'électrodes à la surface des tissus recouvrant la zone contenant le principe, les électrodes étant appliquées sur une même face des tissus, de façon à permettre la délivrance de champs électriques se propageant sous la surface des tissus sur lesquels les électrodes sont appliquées

En complément de certaines des caractéristiques décrites ci-dessus, une caractéristique possible du procédé est qu'un jeu d'électrodes non-invasives est pressée contre la surface des tissus contenant le principe actif afin de modifier leur géométrie pour augmenter le volume de tissus se trouvant entre les électrodes.

En complément de certaines des caractéristiques décrites ci-dessus, une caractéristique possible du procédé est que le générateur est programmé pour émettre alternativement des séquences d'impulsions électriques entre chaque paire d'électrode rapprochée:
- afin d'obtenir un intervalle entre chaque impulsion émise par le générateur dans la zone où se trouvent les tissus contenant le principe actif inférieur à 50 ms
- tout en ayant un intervalle entre deux impulsions au niveau d'une paire d'électrode unitaire supérieure à 100 ms,
- afin d'améliorer l'innocuité des impulsions électriques.

En complément de certaines des caractéristiques décrites ci-dessus, une caractéristique possible du procédé est que la durée entre chaque impulsion électrique émise par le générateur vers une paire d'électrode est comprise ente 1 ms et 50 ms, afin d'améliorer l'innocuité et de diminuer l'intensité des contractions musculaires

En complément de certaines des caractéristiques décrites ci-dessus, une caractéristique possible du procédé est que, dans lequel les impulsions électriques émises par le générateur sont unipolaires et ont une forme carrée.

En complément de certaines des caractéristiques décrites ci-dessus, une caractéristique possible du procédé est que:
- le rapport entre la différence de potentiel entre chaque électrode et leur distance est compris entre 10 Volt/cm et 750 Volt/cm,
- la durée des impulsions électriques est comprise entre 1 et 250 ms,
- la durée entre les impulsions électriques est comprise entre 1 et 1500 ms,
- le nombre d'impulsions de chaque séquence d'impulsions est compris entre 1 et 1000.

En complément de certaines des caractéristiques décrites ci-dessus, une caractéristique possible du procédé est que le rapport Volt/cm appliqué entre les électrodes prend une valeur comprise entre 1,05 et 1,50 fois la valeur optimum des champs pour les tissus ciblés afin d'augmenter le volume de tissus contenant le principe actif traversé par des champs à nombre de Volt/cm optimum

En complément de certaines des caractéristiques décrites ci-dessus, une caractéristique possible du procédé est que on injecte un tranquillisant avant de déclencher les impulsions à l'aide du générateur.

En complément de certaines des caractéristiques décrites ci-dessus, une caractéristique possible du procédé est que le tranquillisant utilisé est la Xylasine.

### Brève description des figures

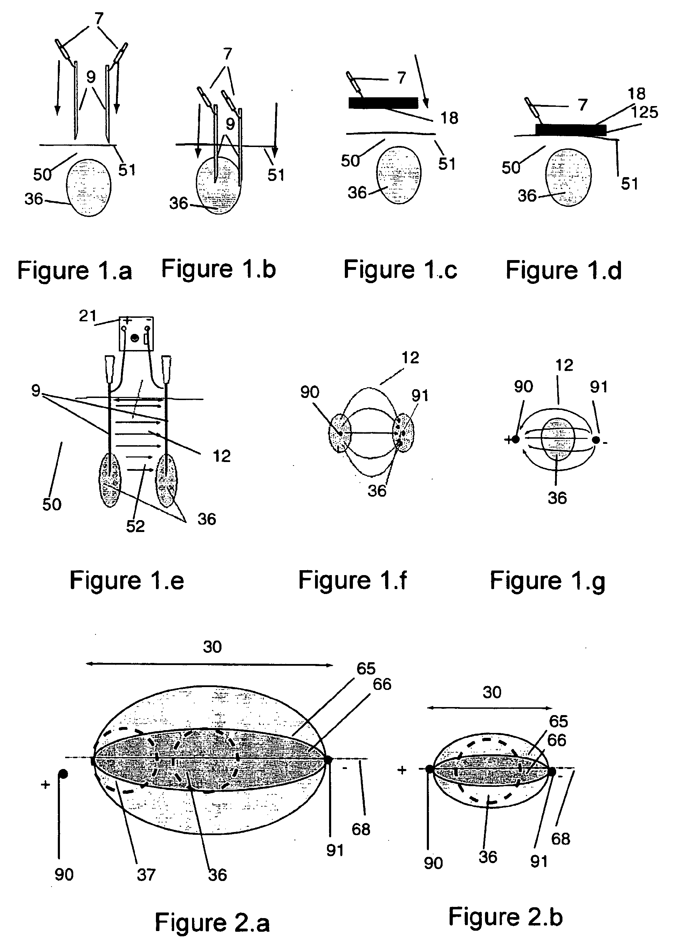

Les figures 1.a et 1.b schématisent la manière de mettre en contact une électrode invasive avec les tissus.
Les figures 1.c et 1.d schématisent la manière de mettre en contact une électrode non-invasive avec les tissus.
Les figures 1.e et 1.f schématisent le procédé d'électrotransfert, avec un dispositif à 2 électrodes, respectivement vue de profil et vue de dessus.
La figure 1.g schématise le procédé d'électrotransfert, le principe actif se trouvant entre les électrodes (vue de dessus).
Les figures 2.a et 2.b schématisent les zones parcourues par des champs, la distance entre les électrodes étant double en figure 2.a, à rapport V/cm constant.
Les figure 3.a, 3.b et 3.c schématisent un exemple de dispositif où les 2 électrodes sont respectivement positionnées à distance, en bordure et à l'intérieur du principe actif
Les figure 4.a et 4.b schématisent un exemple de dispositif avec une électrode positionnée au centre du principe actif et de signe opposé aux autres électrodes bordant le principe actif selon une vue de profil selon un dispositif à 3 électrodes, et selon une vue de dessus, selon un dispositif à 9 électrodes, les électrodes étant toutes de même profondeur.
Les figure 5.a et 5.b illustrent les champs délivrés pour un dispositif avec une électrode placée entre 2 (figure 5.a) ou 4 (figure 5.b) électrodes de signe opposé
Les figures 6.a, 6.b, 6.c et 6.d illustrent les champs délivrés avec un rapport Volt/cm respectivement inférieur, égal, supérieur et très supérieur au rapport optimum pour la nature des tissus traités
Les figures 7.a et 7.b illustrent les champs délivrés pour un dispositif composé de deux paires d'électrodes, les paires électrodes délivrant un rapport Volt/cm égal (figure 7.a) et supérieur de 20% (figure 7.b) au rapport optimum pour la nature des tissus traités
Les figures 8.a et 8.b illustrent un exemple de dispositif composé de 3 électrodes et d'une (figure 8.a) ou deux (figure 8.b) aiguilles d'injection positionnées au centre du dispositif à des profondeurs intermédiaires.
Les figures 9.a, 9.b et 9.c illustrent un exemple de dispositif intégrant une électrode-aiguille d'injection au centre, le principe actif étant respectivement délivré une fois les électrodes enfoncées dans les tissus (figure 9.a), ou délivrés à une profondeur intermédiaire (figure 9.b), les électrodes étant enfoncées jusqu'à la garde (figure 9.c).
Les figures 10.1 et 10.2 illustrent un exemple de dispositif dont les 3 électrodes invasives (respectivement l'électrode centrale) sont isolées électriquement sur une partie à l'aide d'un film plastique.
La figure 10.3 illustre un exemple de dispositif dont l'électrode externe 10 est non-invasive, l'aiguille électrode centrale isolée électriquement sur une partie à l'aide d'un film plastique
La figure 11 illustre un exemple de dispositif d'électrodes solidaires prévu pour être utilisé sans boîtier.
Les figures 12.a à 12.c propose un exemple de pièces composant un dispositif selon l'invention incluant un boîtier et un système de butée
La figure 13 illustre un exemple de pièce d'injection posée dans son habitacle du boîtier.
La figure 14 illustre un exemple d'anneau de verrouillage glissant le long du boîtier fermé.
La figure 15 illustre un exemple de dispositif verrouillé prêt à être appliqué sur le sujet.
La figure 16.a illustre un exemple d'électrode cathéter dont l'aiguille est respectivement dans le cathéter, légèrement déplacée afin de ne pas endommager les tissus ou les parois, et retirée du cathéter.

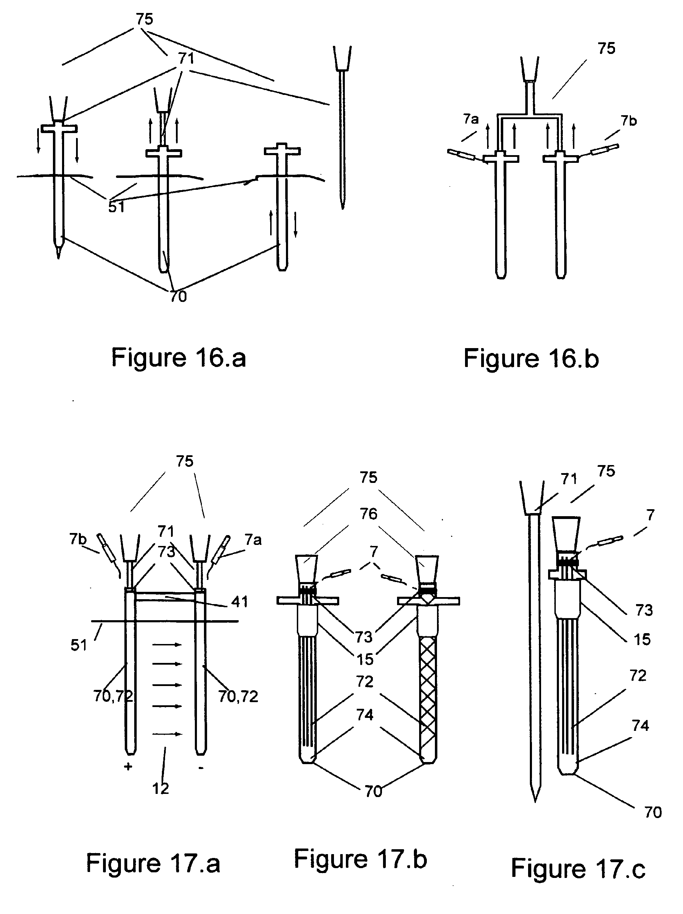

La figure 16.b illustre un exemple d'électrodes cathéter conductrice à double aiguilles, le principe actif étant délivré au travers des cathéters.
Les figures 17.a à 17.c illustrent des exemples d'électrode cathéter conductrice.

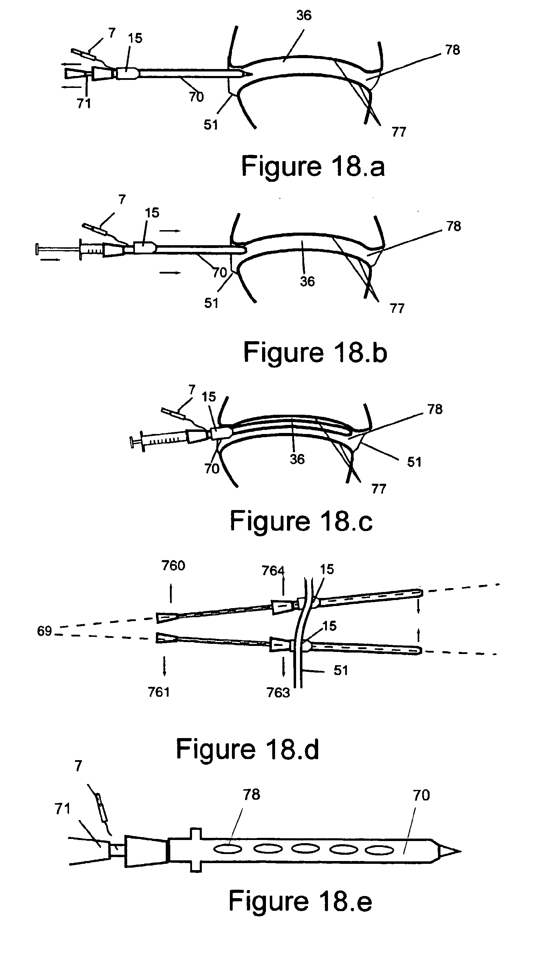

Les figures 18.a à 18.c illustrent un exemple d'utilisation du dispositif d'électrode cathéter conductrice dans une cavité
La figure 18.d illustre un exemple de moyen pour rendre deux électrodes cathéter approximativement parallèles
La figure 18.e illustre un exemple d'électrode recouverte partiellement d'un cathéter non-conducteur

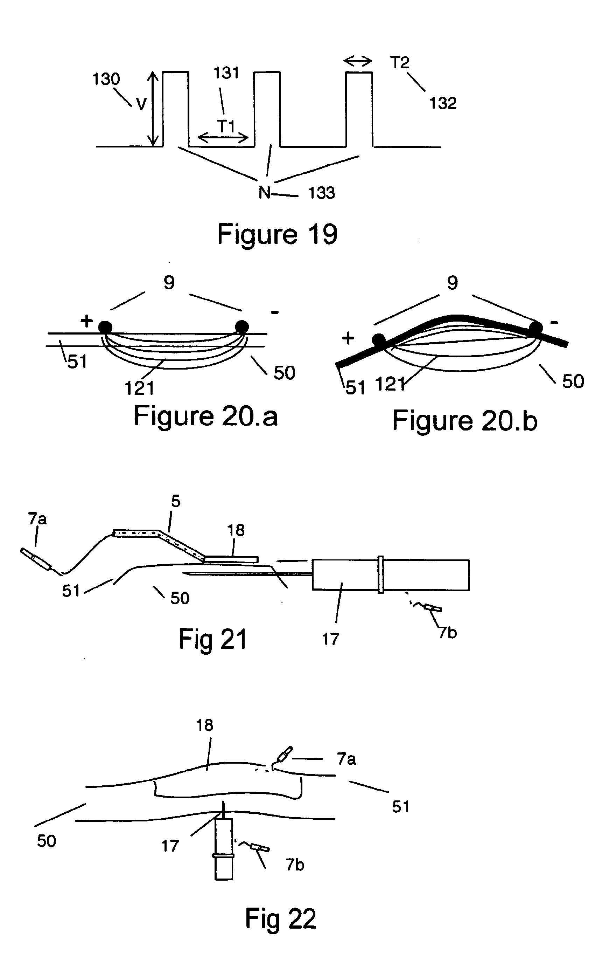

La figure 19 schématise un exemple d'impulsions unipolaires "rectangulaire"
Les figures 20.a et 20.b illustrent les lignes de champs se propageant sous la surface des tissus, entre des électrodes non-invasives avec des électrodes en forme de fil ou de pointe, sur une surface respectivement plane et bombée
La figure 21 illustre un exemple de dispositif composé d'une électrode non-invasive et d'une électrode invasive, cette dernière traversant les tissus afin d'atteindre la zone de tissus, selon un axe approximativement parallèl au plan de l'électrode non-invasive posée sur les tissus.
La figure 22 illustre un exemple de dispositif composé d'une électrode non-invasive et d'une électrode invasive, cette dernière traversant les tissus afin d'atteindre la zone de tissus, selon un axe approximativement perpendiculaire au plan de l'électrode non-invasive posée sur les tissus.

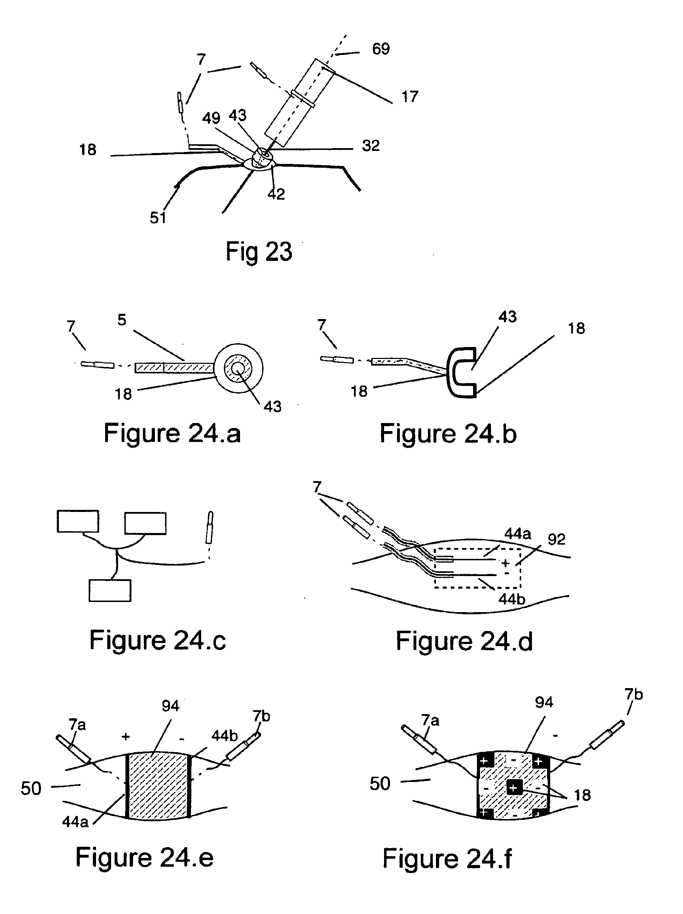

La figure 23 illustre un exemple de dispositif composé d'une électrode non-invasive et d'une électrode invasive, cette dernière traversant l'électrode non-invasive par un orifice, et dont la position et l'angle de l'axe de l'aiguille est guidé par un dispositif de guide.
Les figures 24.a et 24.b illustrent des exemples d'électrodes non-invasives ayant respectivement une forme de disque percé d'un orifice en son centre et de fer à cheval
La figure 24.c illustre un exemple d'électrode non-invasives composé de plusieurs électrodes plates non solidaires
La figure 24.d illustre un exemple de dispositif composé de deux électrodes filaires parallèles maintenus par une bande adhésive
La figure 24.e illustre un exemple de dispositif composé de deux électrodes filaires maintenues par une gaine
La figure 24.f illustre un exemple de dispositif composé d'un ensemble d'électrodes non-invasives disposés en damier et maintenus par une gaine

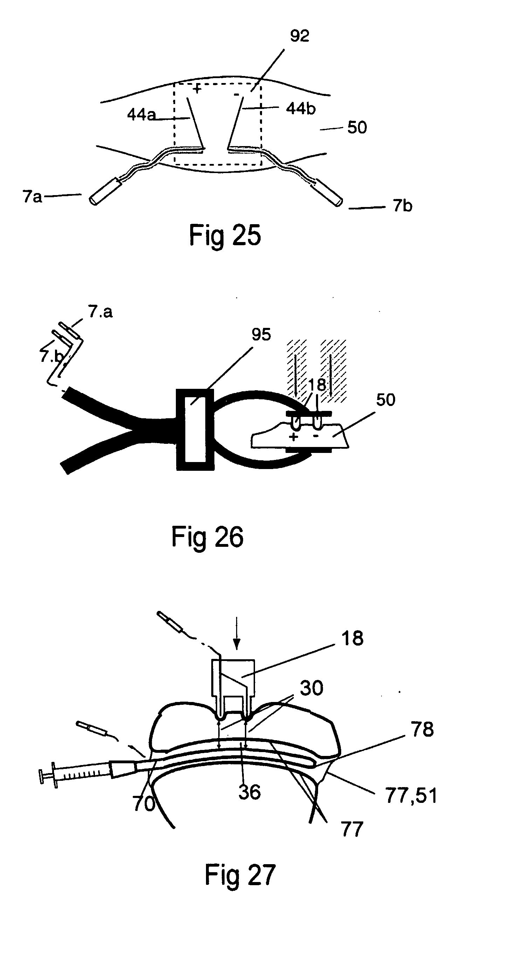

La figure 25 illustrent un exemple de dispositif composé d'électrodes non-invasives filaires suivant un axe non parallèle
La figure 26 illustrent un exemple de dispositif composé d'électrodes non-invasives rendues solidaire par une pince permettant de presser et de déformer la géométrie des tissus
La figure 27 illustrent un exemple de dispositif composé de deux électrodes non-invasives solidaires et reliées à la même borne du générateur, étant pressées contre les tissus afin de se rapprocher d'une électrode cathéter reliée à l'autre borne du générateur et se trouvant dans une cavité.

### Présentation de l'invention

La présente invention permet d'injecter une solution de principe actif dans tous les tissus, notamment dans le muscle, les tumeurs, les articulations, le derme et l'épiderme, et dans tous les organes, à l'exception du coeur, et notamment la vessie, l'estomac, les reins, les poumons, tous les organes de la tête, incluant en particulier le cerveau, les oreilles, l'oeil, la gorge, d'un animal ou humain vivant ou non et de délivrer les champs afin de perméabiliser les tissus et de favoriser le transfert du principe actif dans les cellules.

Le dispositif d'électrodes de l'invention est composé:
- d'un premier ensemble d'électrodes 11 composé d'au moins une électrode, les électrodes étant reliées à une borne d'un générateur d'impulsions électriques 21
- d'un deuxième ensemble d'électrodes 10 composé d'au moins une électrode, les électrodes étant reliées à l'autre borne du générateur d'impulsions électriques 21, et donc d'une tension différente du premier ensemble d'électrodes.
- d'un moyen d'injecter le principe actif 36

Le moyen d'injecter le principe actif peut être composé d'une ou plusieurs aiguilles. Il peut aussi s'agit d'un moyen d'introduire le principe actif sous pression, à l'aide, par exemple, d'un pistolet à pression, par patch ou tout autre moyen de l'homme de l'art.

Dans la présente demande, l'expression "électrode" désigne tout type d'électrode, notamment pleine ou creuse, invasive ou non-invasive. Le terme "électrode invasive" désigne une électrode conçue pour pénétrer à l'intérieur des tissus. Le terme "électrode non-invasive" désigne une électrode conçue pour rester à la surface des tissus.

Les deux ensembles d'électrodes 11, 10 sont composés d'électrodes invasives et/ou d'électrodes non-invasives.

Comme exemplifié en figures 1.a à 1.d, dans la présente demande, par "mettre en contact une électrode avec les tissus à traiter", on entend tout d'abord: quand l'électrode est invasive (9), l'enfoncer dans les tissus (50) du sujet. Elle peut être enfoncée à l'intérieur de la zone de tissus devant contenir le principe actif, et peut aussi être enfoncée en bordure, voir à proximité. Sinon, quand l'électrode est non-invasive (18), on entend: poser l'électrode à la surface des tissus concernés (51).On peut aussi insérer une couche de gel conductrice (125) entre l'électrode et les tissus pour améliorer la conductivité. Dans les deux cas, l'électrode est en contact électrique avec les tissus. Dans le cas d'une pluralité d'électrodes mises en contact avec les tissus à traiter, celles-ci peuvent être, selon leur forme, enfoncées dans les tissus ou posés à la surface des tissus de manière successive ou simultanée. Mettre en contact une électrode avec la zone des tissus à traiter sous-entend que le principe actif peut être injecté avant, pendant ou après que l'électrode est mise en contact avec les tissus. Dans le cas d'une pluralité d'électrodes, le principe actif peut être injecté en totalité ou partiellement avant, pendant ou après que chaque électrode est mise en contacte avec les tissus. L'électrode est reliée au générateur par un fil électrique (7) ou tout autre moyen connu de l'homme de l'art.

Les impulsions électriques délivrées peuvent avoir une forme carrée, unipolaire, bipolaire, exponentielle ou autre. Le générateur génère pour chaque paire d'électrode une ou plusieurs séquences prédéfinies d'impulsions électriques, dont le nombre est inférieur à 100, chaque impulsion est de préférence caractérisée par les éléments suivants qui peuvent varier entre deux impulsions, ou rester identiques au sein d'une même séquence:
- Rapport Volt/cm (différence de tension / distance entre les deux électrodes) compris entre 10 V/cm et 1 500 V/cm
- La différence de tension entre chaque paire d'électrodes peut être de 1 V à 2 500 V
- Durée de l'impulsion, comprise entre 1 et 1000 ms
- Durée entre deux impulsions comprise entre 1 et 2000 ms
- distance séparant les électrodes dans la zone où le principe actif est injecté inférieure à 10 cm

La durée entre deux séquences d'impulsion est inférieure à 600 sec, et le nombre de séquences est inférieur à 25. L'intensité délivrée sera inférieure à 5 Ampères.

Les électrodes invasives et non-invasives sont composées de matériaux métalliques de préférence inoxydables et de qualité médicale. Elles peuvent être pleines ou creuses, par exemple des aiguilles d'injection médicales.

Un exemple de dispositif à 2 électrodes invasives 90,91 est décrit en figure 1.e et 1.f.

Pour une efficacité optimum pour l'électrotransfert, le principe actif doit se trouver intégralement traversé par les champs 12, et donc se trouver entre les électrodes, comme représenté en figure 1.g pour un dispositif à 2 électrodes invasives 90,91. Comme représenté en figure 2.a, la zone centrale 36 est mieux couverte par les champs qu'une zone excentrée 37, ceci pour des raisons évidentes de géométrie. Le principe actif doit de préférence se trouver au centre du dispositif d'électrodes.

L'efficacité de l'électrotransfert dépend de la valeur des champs, défini par le rapport entre la tension entre électrodes et la distance 30 entre les 2 pôles (électrodes), et dont l'unité est: Volt/cm. Ce rapport doit se rapprocher d'une valeur optimum dépendant de la nature des tissus, et plus particulièrement de la taille des cellules composant les tissus. Cette valeur est appelée dans la présente demande "Champ Optimum Direct". Les tissus 66 parcourus par les champs selon ce rapport sont représentés en gris-foncé dans les schémas. A un rapport inférieur, l'efficacité tend à diminuer car la force des champs devient trop faible. A un taux supérieur, les gains sont contrebalancés par la toxicité de l'hyperperméabilisation des cellules entraînant la mort cellulaire, et l'efficacité tend alors à diminuer. Les tissus 65 parcourus par les champs selon ce rapport moins optimum (donc du à un rapport Volt/cm trop élevé ou trop faible) sont représentés en gris-clair dans les schémas de la présente demande.

Les champs délivrés 12 entre deux électrodes occupent un volume ayant une forme approximativement ovale selon l'axe 68 reliant les deux électrodes. Plus les lignes de champs s'éloignent de cet axe, plus la distance parcourue est importante, et donc plus le rapport Volt/Cm diminue.

La distance entre les électrodes doit être minimum pour des raisons d'innocuité. En effet, une grande distance implique:
- L'accroissement de manière proportionnelle de la tension entre les électrodes (90,91) pouvant atteindre alors des seuils dangereux.
- La diffusion du courant sur une surface plus importante. Ceci est illustré par la figure 2.a, où la tension et la distance entre les électrodes est doublée par rapport à la figure 2.b. Le courant traverse plus de surface innervée, entraînant plus de contraction, et engendrant une réaction douloureuse plus intense.

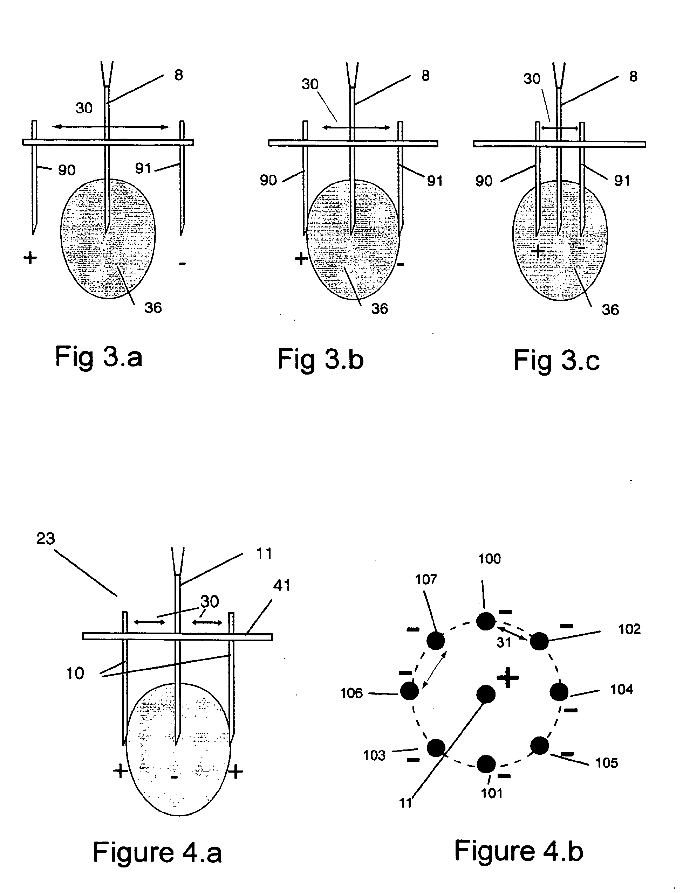

Pour avoir une distance 30 entre les électrodes minimum, tout en préservant l'efficacité (maximum de principe actif traversé par les champs) les électrodes doivent border le principe actif injecté 36. Ceci est illustré par les es figures 3.a à 3.c, exemplifiant un dispositif dans lequel l'aiguille d'injection 8 du principe actif se trouve centrée entre les deux électrodes invasives 90,91.

L'invention propose un dispositif où le premier ensemble d'électrodes 11 est introduit au sein du principe actif, de préférence en son centre, et est entouré par le deuxième ensemble d'électrodes 10. Un exemple est illustré en figure 4.a avec des électrodes invasives. La distance 30 entre les électrodes est divisée de moitié par rapport au dispositif précédent (figure 3.b). Si les électrodes du deuxième ensemble sont non-invasives, alors le deuxième ensemble d'électrodes et positionné à la surface des tissus, sur le site d'injection du principe actif.

Dans une forme préférée de l'invention, si le dispositif est constitué de plus d'une électrode invasive, alors les électrodes invasives ont toutes la même profondeur.

Afin d'améliorer l'efficacité du transfert de principe actif dans les cellules, les électrodes du deuxième ensemble 100 à 107 doivent être réparties régulièrement en formant un cercle dont les électrodes centrales 11 forment le centre. Elles doivent donc se trouver à distance constante 31 les unes des autres, comme illustré en figure 4.b.

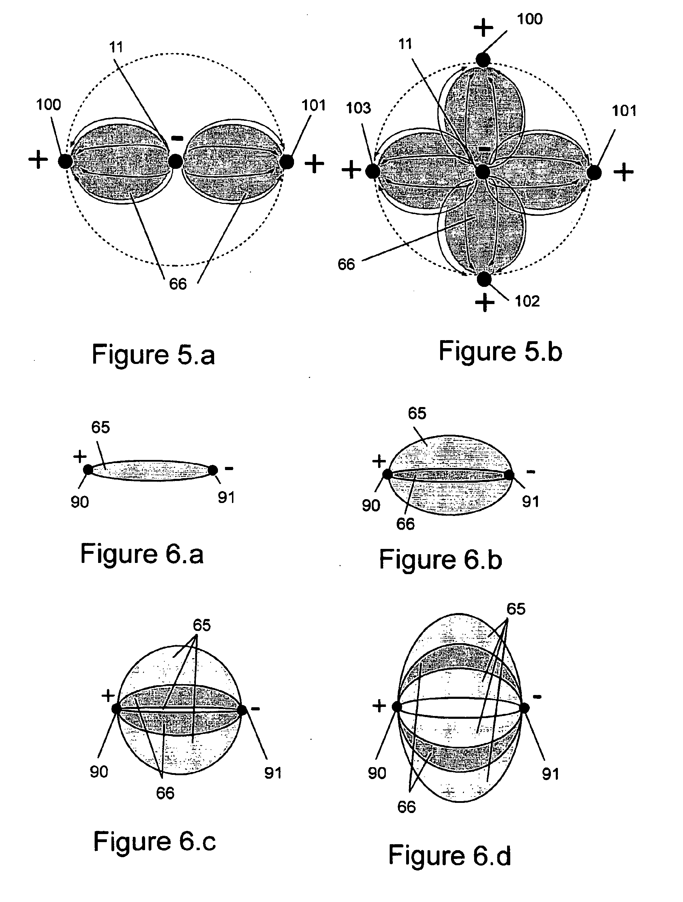

Les figures 5.a et 5.b sont des formes préférées de l'invention, pour des électrodes invasives ou non-invasives et illustrent la surface électrotransférée avec un dispositif où le deuxième ensemble d'électrodes est composé respectivement à 2 électrodes (100, 101) et à 4 électrodes (100 à 103). Les champs délivrés par le dernier dispositif couvrent une surface plus importante, proche de la sphère.

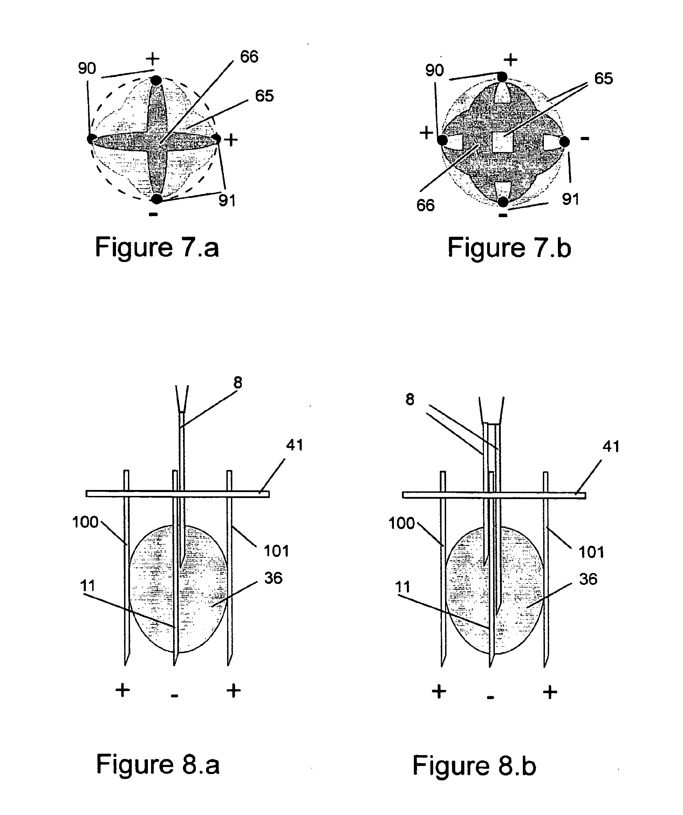

La localisation des zones traitées avec une efficacité optimum 66, et avec une efficacité moyenne 65, dépend du rapport Volt/cm appliqué sur la paire d'électrodes:
- En figure 6.a, le rapport Volt/Cm entre les électrodes est inférieur au Champ Optimum Direct.
- En figure 6.b, le rapport Volt/Cm entre les électrodes est égal au Champ Optimum Direct.
- En figure 6.c le rapport Volt/Cm est légèrement supérieur au Champ Optimum Direct, et donc une zone plus importante se trouvant entre les électrodes est traitée avec efficacité optimum 66, les zones limitrophes avec efficacité moyenne 65.
- En figure 6.d les champs sont supérieurs au Champ Optimum Direct, et leur toxicité importante annihile les effet de l'électrotransfert sur les tissus placés sur l'axe entre les deux électrodes.
- On constate ainsi que l'efficacité la meilleure est obtenue si le rapport Volt/cm appliqué entre les électrodes est légèrement supérieur (exemplifié aussi en figure 7.b, pour un rapport compris entre 105% et 150 %) au Champ Optimum Direct par rapport aux résultats obtenus en appliquant au Champ Optimum Direct entre les électrodes (exemple figure 7.a). Cette méthode ne peut cependant n'être appliquée que si l'accroissement du champ électrique en Volt/cm reste tolérable pour le sujet sur le plan de l'innocuité et de la toxicité.

Dans un dispositif à plusieurs paires d'électrodes invasives localisées dans la même zone (exemplifié figures 7.a et 7.b pour un dispositif à 2 paires), les séquences d'impulsion peuvent être délivrées de manière successive, simultanée ou imbriqués. Le

Dans le cas d'un dispositif où le deuxième ensemble d'électrodes est composé d'au moins 2 paires d'électrodes, l'efficacité est aussi améliorée si les paires d'électrodes transmettent successivement les champs, par rapport à une délivrance simultanée des champs, certains tissus étant traités 2 fois.

Afin de positionner précisément le premier ensemble d'électrodes au centre du principe actif, l'aiguille d'injection 8 du principe actif ainsi que les électrodes 11,100,101 peuvent être maintenues entre-elles à l'aide d'une ou plusieurs pièces de sertissage 41, qui permettent le maintien dans une géométrie définie. Ceci est exemplifié en figure 8.a pour un dispositif composé d'électrodes invasives.

Dans la suite du descriptif, sauf indication contraire, les électrodes du premier ensemble d'électrodes 11 sont invasives. Les exemples illustreront des dispositifs complets ou étant une partie d'un dispositif contenant plus d'électrodes. Les électrodes-aiguilles invasives du premier ensemble d'électrodes seront représentées dans les schémas par une seule électrode-aiguille 11. Le premier ensemble d'électrodes se trouvant au centre du dispositif, et incluant le moyen d'injection du principe actif sera nommé parfois: "électrode Première", le deuxième ensemble d'électrodes invasives sera nommé parfois: "électrodes externes".

Afin que le principe actif soit bien situé entre les électrodes, de manière exemplifiée en figure 8.a sur un dispositif de 3 électrodes invasives, l'aiguille d'injection 8 doit avoir une profondeur inférieure à celle des électrodes centrales et/ou des électrodes externes. La figure 8.b exemplifie un dispositif à plusieurs aiguilles d'injection 8, positionnées contre l'électrode aiguille centrale 11, et disposées régulièrement à des profondeurs inférieures à celles des électrodes afin de répartir uniformément le principe actif entre les électrodes. On peut remarquer que les aiguilles d'injection, étant en contact avec l'électrode centrale, font aussi office d'électrode de fait. Dans cet exemple, les électrodes et aiguilles sont rendues solidaires à l'aide d'un support (41)

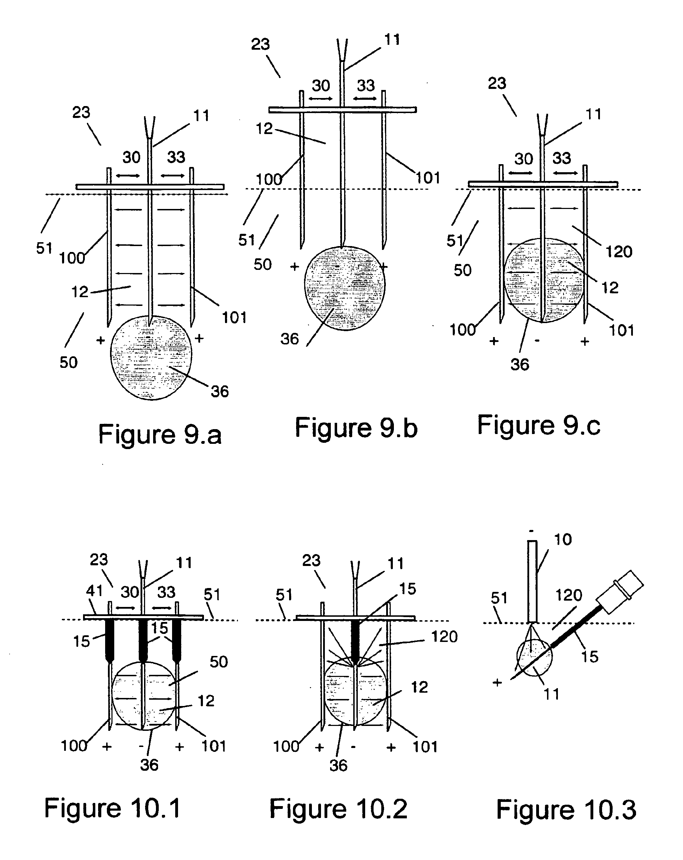

L'électrode centrale peut faire aussi office d'aiguille d'injection. Cependant, l'inconvénient de ce dispositif est qu'une partie du principe actif est diffusé en dessous l'électrode aiguille centrale, et n'est plus traversée par les champs (figure 9.a). Une méthode proposée par l'invention est d'injecter le principe actif 36 à une ou plusieurs profondeurs intermédiaires dans les tissus (50). Le dispositif d'électrodes 23 est enfoncé à chaque étape plus profondément dans les tissus, afin d'injecter le principe actif. A la dernière étape, les électrodes sont enfoncées jusqu'à la garde, sans délivrer de principe actif. Cette méthode permet ainsi d'injecter le principe actif dans une zone qui sera positionnée de manière optimale entre les électrodes. Cette méthode est exemplifiée figure 9.b et 9.c pour un dispositif d'électrodes invasives (100,101,11), et avec une seule étape intermédiaire où tout le principe actif est injecté.

Par l'injection en profondeur du principe actif, notamment dans le muscle du grand mammifère ou de l'homme, des champs 120 sont aussi diffusés dans des tissus n'hébergeant pas de principe actif. Ceci est illustré figure 9.c. Or, cela entraîne une toxicité et une contraction ou douleur du sujet inutile et parasite. L'invention propose d'appliquer un isolant électrique 15 à la partie supérieure des aiguilles 100,101,11, comme exemplifié en figure 10.1 pour un dispositif à 3 électrodes invasives, ce qui limite fortement la quantité de courant traversant les tissus. Cet isolant a aussi pour effet bénéfique d'isoler les électrodes de la surface de la peau, afin d'éviter que les champs ne se propagent juste sous la peau 51 du sujet. Le fait de n'isoler que les aiguilles rattachées à une seule borne du générateur ne diminuera qu'en partie les courants parasites 120 comme illustré en figure 10.2 pour un exemple de dispositif à 3 électrodes invasives. Il peut aussi être intéressant d'isoler la partie supérieure de l'aiguille électrode centrale pour un dispositif comprenant une ou plusieurs électrodes non-invasives (un exemple de dispositif est illustré figure 10.3).

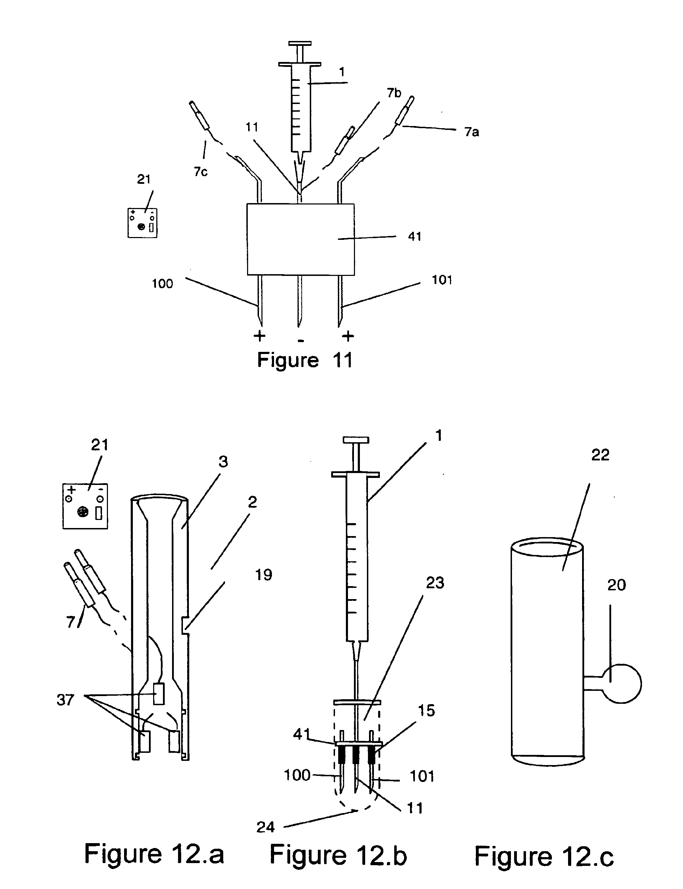

Les aiguilles et les électrodes invasives ou non-invasives peuvent être rendues solidaires et serties par un support rigide ou légèrement flexible (41). Les composants invasifs seront alors parallèles. Un exemple est illustré en figure 11 pour un dispositif d'aiguilles invasives (100,101), les électrodes et électrodes-aiguilles traversant un bloc isolant électriquement 41, tout en étant directement relié au générateur 21 à l'aide de fil électrique 7a,7b.

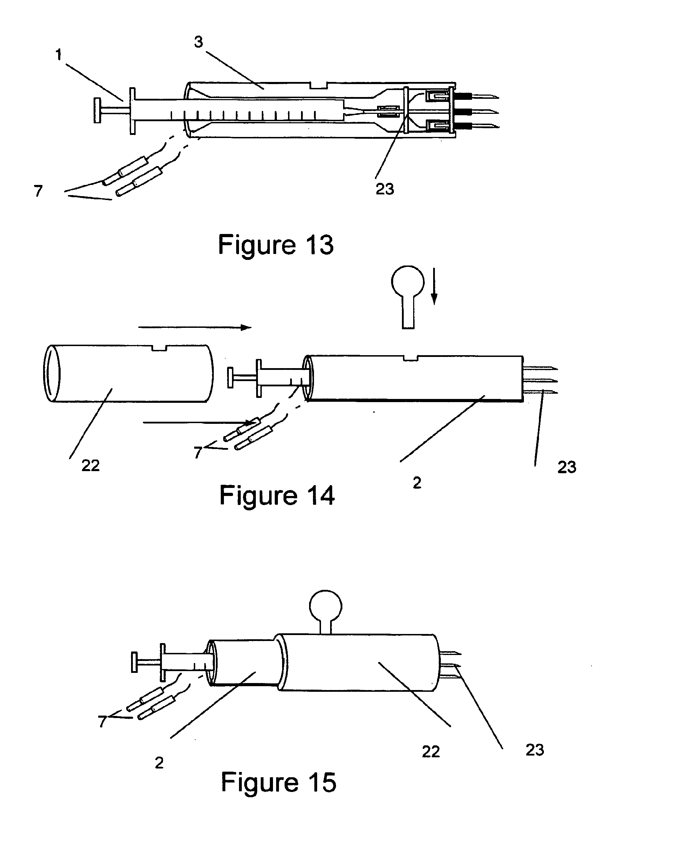

Dans l'optique d'électrodes jetables et économiques, le dispositif pourra en outre comprendre un boîtier 3 permettant de relier les électrodes au générateur. Ce boîtier permettra une prise en main du dispositif d'électrodes aisément manipulable et assure l'isolation du manipulateur vis-à-vis des courants électriques. Le boîtier pourra intégrer, en outre, le moyen d'injection du principe actif et son réservoir 1. De plus, il pourra permettre d'enfoncer le dispositif d'électrodes à des profondeurs pré-définies, par exemple à l'aide de butées. A chaque étape, sauf la dernière, il permettra d'injecter une partie du principe actif qui sera répartie de manière homogène entre les électrodes (ou entre la partie non isolée des électrodes). Un exemple de dispositif d'électrodes jetables 23, de boîtier 3 avec son système de butée 20-22 et présenté en figures 12.a, 12.b et 12.c. La méthode d'assemblage de cet exemple de boîtier et des électrodes et de sa butée est présenté figures 13 à 15.

Les électrodes invasives doivent être adaptées pour le traitement de zones fragiles afin de ne pas blesser la zone traitée avec l'aiguille lors de la contraction musculaire provoquée par la délivrance des champs (comprenant les tissus traversés par l'électrodes et les parois 77 bordant ces tissus). En effet, certains tissus et parois ne se reconstituent pas ou peu, et il faut donc éviter de les blesser pendant les manipulations de positionnement des électrodes et d'injection du principe actif et de délivrance des champs. Le dispositif de l'invention, appelé "électrode cathéter" dont un exemple est illustré figures 16.a, 16.b, 17.a à 17.c, permet d'éviter ce risque. La figure 17.c décrit tous les composants d'une électrode cathéter 75. Le dispositif est adapté pour tous tissus, notamment le muscle, et est particulièrement indiqué pour organes composés d'une cavité fermée 78 contenant des matériaux fluides, mêmes visqueux. Ce dispositif est particulièrement indiqué pour le traitement de la cavité synoviale de toute articulation des membres de tout mammifères de plus de 2 kg, y compris l'être humain.

Une électrode Cathéter est une électrode invasive, composée d'une aiguille invasive 71 recouverte d'un cathéter 70. Un cathéter est un tube composé de matériaux fins, flexibles et plus ou moins résistants à des forces de compression suivant son axe, par exemple du silicone. Il est prévu pour être introduit dans les tissus à l'aide de l'aiguille 71 placée à l'intérieur du cathéter. La surface du cathéter est rendue conductrice électriquement, tout en lui conservant une certaine souplesse. Une fois l'électrode cathéter dans les tissus, l'aiguille est reculée partiellement, voir entièrement retirée du dispositif. Le cathéter 70, ne peut plus alors détériorer les tissus. Si les tissus sont fluides, il peut être enfoncé et dans certains cas ajusté au niveau de sa direction.

Dans une variante de l'invention, le principe actif peut être introduit simultanément dans plusieurs électrodes composant le dispositif, comme exemplifié figure 16.b par un exemple d'électrodes cathéter conductrice à double aiguilles, le principe actif étant délivré au travers des cathéters, les deux électrodes étant solidaires..

Dans une variante de l'invention, les électrodes cathéter peuvent être composés de plusieurs aiguilles et cathéters. Les cathéters peuvent êtres alignés, être disposés sous forme de cercle ou prendre toute autre disposition. Un exemple est illustré figure 17.a, où les deux cathéters sont chacun reliés à une borne différente du générateur, le support 41 assemblant les électrodes et n'étant pas conductrice électriquement, les aiguilles étant indépendantes.

Pour être conducteur, le cathéter doit être recouvert de film ou de fils métalliques ou tous autres matériaux conducteur électriquement 72. Les fils peuvent prendre toute disposition (tressés, parallèles, maillés...) prévue par l'homme de l'art.

Si le dispositif est constitué de plus d'une électrode cathéter, alors les électrodes peuvent être rattachées à un même support ou être indépendantes.

Dans la méthode exemplifiée figure 18.a à 18.d, le dispositif peut est légèrement introduit dans la cavité grâce aux aiguilles invasives 71 qui sont ensuite retirées. Le principe actif est ensuite injecté dans la cavité par l'intérieur du cathéter 70. Il peut être injecté au fur et à mesure que l'électrode est enfoncée dans la cavité. Dans le cas d'une cavité synoviale, le cathéter étant souple permet de plier l'articulation afin de bien répartir le principe actif.

Le principe actif peut être délivré en une seule fois ou en plusieurs fois, ou de manière continue, au fur et à mesure qu'on enfonce le cathéter dans les tissus.

Dans une variante de l'invention, l'aiguille 71 de chaque cathéter 70 est légèrement déplacée, de façon à ne plus pouvoir rentrer en contact avec les parois de la cavité. L'aiguille reste cependant dans le cathéter, de façon à le rendre plus rigide pendant qu'il est enfoncé dans la cavité. Dans ce cas, le principe actif peut être injecté au fur et à mesure par l'aiguille 71. Une fois l'ensemble positionné, les aiguilles 71 peuvent être retirées avant la délivrance des champs.

Quand le générateur délivre les impulsions électriques, le courant passant par la surface métallique des cathéters.

Les cathéters peuvent être recouverts, dans leur partie supérieure, d'un isolant électrique 15 (exemplifié figure 17.b à 18.d), afin que les champs ne se propagent que dans la cavité. Dans ces conditions, si les parois de la cavité ne sont pas innervées, alors le sujet ne ressent pratiquement pas de douleur et n'a qu'une légère contraction musculaire, et des voltages importants peuvent être délivrés.

Les électrodes cathéter peuvent être utilisées sur tous types de tissus. Sur des tissus non fluides, et notamment les muscles, l'aiguille ne doit pas être enlevée avant que le dispositif ne soit enfoncé jusqu'à la profondeur finale souhaitée.

Si le dispositif est composé d'au moins 2 cathéters reliés à des bornes différentes du générateur, alors il est préférable que les axes des cathéters soient parallèles. Les cathéters peuvent être rendues solidaires à l'aide d'un support ou par l'usage d'un boîtier. Cependant, l'usage d'électrodes cathéters solidaires peut être difficile pour des tissus difficilement accessibles. Il est possible, une fois la première électrode posée, d'utiliser un guide forçant le parallélisme de la deuxième électrode: le guide est appliqué sur la partie émergente de l'aiguille enfoncée pour guider, par un autre orifice du guide l'électrode suivante. Ce principe peut être appliqué pour tout type d'aiguilles invasives.

Beaucoup de méthodes et dispositifs permettent de connaître précisément la distance entre les extrémités (74) des électrodes. Cela peut être visualisé par des systèmes d'imagerie médicale. Une autre technique est d'insérer des électrodes aiguilles non invasives à l'intérieur des cathéters et en les utilisant comme électrode pour lancer une légère impulsion par leur pointe qui dépasse le cathéter. Ceci permet de connaître la résistance entre les électrodes, donc la distance.

Il est possible aussi d'utiliser un dispositif afin de régler le parallélisme. On peut utiliser, par exemple, un dispositif composé de tiges rigide 760, 761 que l'on introduit dans les cathéter afin d'orienter les cathéter comme exemplifié en exemple figure 18.d, ou un appareil adapté pressant les extrémités extérieur (76) des électrodes 763, 764. Un boîtier peut aussi être utilisé, qui en outre peut assurer le contact électrique entre les électrodes cathéter et le générateur.

Le générateur pourra contrôler la quantité d'intensité émise pendant la délivrance des champs, et par là renseigner la qualité de la réussite de l'acte, par comparaison à des abaques pré-enregistrées ou au résultat de la première impulsion émise.

Le dispositif peut comporter en complément des électrodes cathéter, une ou plusieurs électrodes non invasive posées surface des tissus bordant la cavité, reliée à l'une des bornes du générateur, sachant qu'au moins une électrode cathéter est liée à l'autre borne. Cette électrode peut être non-invasive (par exemple une pastille positionnée sur un genou). Le dispositif peut comporter en complément des électrodes cathéter une électrode invasive (par exemple une aiguille permettant d'atteindre la périphérie de la hanche) mais se rapprochant uniquement de la zone de tissus contenant le principe actif, sans la pénétrer. Dans ce dernier cas, cette aiguille pourra être recouverte d'un isolant électrique sauf à son extrémité. Si le dispositif comporte plusieurs paires d'électrodes, les impulsions entre chaque paire peuvent être délivrées simultanément ou successivement.

Les électrodes cathéter peuvent aussi être percées d'orifices (78) dans la paroi du cathéter (70), celui-ci ne nécessitant plus de couverture conductrice. Un exemple est illustré figure 18.e. Dans ce cas, c'est l'aiguille (71) qui assure le contact électrique avec les tissus à traiter, en étant en contact direct avec les tissus au travers des orifices. Avec ce type d'électrode, il est aussi recommandé, pour des raisons d'innocuité, de légèrement déplacer l'aiguille à l'intérieur du cathéter avant de déclencher les champs. Ce type d'électrode est appelé dans le présent descriptif: électrode recouverte partiellement d'un cathéter non conducteur.

Dans le présent descriptif, par le terme: électrode cathéter, on entend tous types d'électrodes cathéter; notamment les électrode recouvertes partiellement d'un cathéter non conducteur et les électrodes cathéter conductrices.

La suite de la description prend en compte tous types d'électrodes et d'assemblage d'électrodes.

L'utilisation d'émission par le générateur d'impulsions de test à faible tension et faible durée avant la délivrance des impulsions thérapeutiques peut être généralisée à tous types d'électrodes. Ces impulsions permettent de contrôler la distance des électrodes en analysant l'intensité de courant engendrée, et en tenant compte de la nature des tissus et de la géométrie des électrodes. Cela permet aussi de recalculer le programme d'impulsions des séquences. Le programme de séquences peut aussi être recalculé en temps réel par moyen informatique pendant délivrance des champs, afin de corriger la suite de la séquence. Cela peut être utile, par exemple si l'écartement entre les électrodes varie du fait de la contraction musculaire.

Afin d'améliorer l'innocuité du dispositif, l'utilisation de tranquillisants comme la Xylasine (par exemple Xylasine 7 mg/ml) diminue très fortement la contraction des muscles et la réaction de douleur provoquée par la délivrance des champs, et correspond à un moyen alternatif notamment quand les anesthésiques constituent une contre-indication.

Les séquences d'impulsions sont définies par les caractéristiques et paramètres suivants:
- forme des impulsions : rectangulaire - unipolaire exemplifié par la figure 19, sinusoïdale, bipolaire, exponentielle, etc....
- la différence de tension 130 entre les électrodes, le nombre d'impulsions 133, durée de chaque impulsion 132
- intervalle de temps entre chaque impulsion 131.

L'inventeur a constaté que l'utilisation d'un intervalle de temps entre deux impulsions électriques de petite durée (comprise en 1 et 100 ms) diminuait très fortement la contraction des muscles et la réaction de douleur provoquée par la délivrance des champs, comparé aux valeurs usuellement pratiquées (supérieures de 100 ms). Cette diminution de la contraction musculaire et de l'apparente réaction de douloureuse est améliorée au fur et à mesure de la diminution de l'intervalle.

Les expériences réalisés sur des chiens et des chats ont montré les points suivants:
- L'usage d'un tranquillisant entraîne une très nette diminution de la douleur et des contractions musculaires,
- L'utilisation d'un intervalle de temps compris entre 1 et 50 ms entraîne une très nette diminution de la douleur et des contractions musculaires, cette diminution étant plus importante à fur et à mesure que la durée diminue.

Cependant, des intervalles très courts peuvent entraîner une baisse d'efficacité de l'électrotransfert. Il est alors possible d'utiliser un dispositif à plusieurs paires d'électrodes, proches, parallèles ou entrecroisées, le générateur alternant le déclenchement de chaque impulsion. Dans la présente demande, les impulsions seront nommées "impulsions imbriquées".

Cela permet, au niveau global de la zone de tissus, de simuler une division de la durée entre deux impulsions par le nombre de paires d'électrodes mises en oeuvre. Il est recommandé de décaler les impulsions d'une durée égale à la durée de l'intervalle entre les impulsions divisé par le nombre de paires. Une même électrode peut être associée à plusieurs électrodes rattachées à une autre borne du générateur pour réaliser plusieurs paires d'électrodes. Par exemple, dans le cas d'un dispositif à 3 électrodes (ex figure 4.a, 5.b), chaque paire correspond au couple: électrode centrale, une des électrodes externes.

Dans un mode préféré de l'invention, tout ou partie des électrodes du deuxième ensemble d'électrodes sont non-invasives, et disposées à la surface des tissus recouvrant (ou proche de) la zone contenant le principe actif contenant le principe actif.

Les électrodes non-invasives peuvent être solidaires des électrodes invasives, ou indépendantes Les deux ensembles d'électrodes peuvent être constitués d'électrodes invasives et/ou non-invasives, les électrodes non-invasives étant disposées à la surface des tissus recouvrant la zone contenant le principe actif.

Les deux ensembles d'électrodes peuvent aussi n'être constituées que d'électrodes non-invasives. Dans ce cas, dans un mode préféré de l'invention, les électrodes sont appliquées sur une même face des tissus 51 contenant le principe actif de façon à permettre la délivrance de champs 12 se propageant sous la surface des tissus sur lesquels les électrodes 9 sont appliquées. Les champs délivrés se propagent entre les électrodes travers d'une couche de tissus intermédiaires (peau, graisse...) et traversent le tissus contenant le principe actif comme exemplifié en exemple figure 20.a et 20.b

Les électrodes non-invasives peuvent être solidaires ou indépendantes; elles peuvent être à usage unique, ou réutilisables.

L'invention propose un dispositif qui comporte une ou plusieurs électrodes invasives reliées à la première borne du générateur et une ou plusieurs aiguilles non-invasives reliées à la deuxième borne du générateur, ces électrodes n'étant pas solidaires. Les électrodes invasives peuvent traverser les tissus 50 afin d'atteindre la zone contenant le principe actif. Un un exemple est illustré figure 21 où l'axe de l'électrode invasive 17 est sensiblement parallèle à au plan suivi par surface de l'électrode non-invasive 78 ou par la surface des tissus 51. Un exemple est illustré figure 22 où l'axe de l'électrode invasive 17 est approximativement perpendiculaire 18 au plan suivi par la surface de l'électrode non-invasive ou par la surface des tissus. Dans une forme préférée de l'invention, un guide 49 peut permettre de positionner de manière précise l'emplacement où l'électrode invasive doit pénétrer les tissus et/ou l'angle de pénétration de l'axe 69 de l'électrode invasive (exemplifié figure 23).

La surface de chaque électrode non-invasive 18 en contact avec les tissus au moment de la délivrance des champs peut avoir toute forme. Elle peut représenter toute géométrie: rectangle, triangle, cercle (exemplifié figure 24.a), demi-cercle, arc de cercle, disque, fer à cheval (exemplifié figure 24.b), ovale, filaire (exemplifié figure 24.d), symétrique ou asymétrique... Chaque électrode peut disposer d'un orifice 43 (dont un exemple est illustré figure 24.a et 24.b), permettant aux électrodes invasives de la traverser. La figure 24.c illustre un exemple d'électrode non-invasives composé de plusieurs électrodes plates non solidaires

Les électrodes non-invasives peuvent compléter un dispositif d'électrodes invasives et d'électrodes cathéter.

La surface de chaque électrode non-invasive en contact avec les tissus au moment de la délivrance des champs peut avoir toute taille. Elle peut être très petite, comme une pointe légèrement aplatie à son extrémité. Elle peut être plus grande, permettant par exemple de recouvrir de manière significative la surface des tissus contenant le principe actif, ou de recouvrir une surface plus importante.

Chaque électrode non-invasive peut être rigides ou souples. Chaque électrode invasive peut être plate ou légèrement courbes afin d'épouser la forme de la surface des tissus cibles.

Chaque électrode non-invasive peut être maintenue contre les tissus à l'aide d'un manche 5, d'un boîtier, d'une gaine élastiques 94 (dont un exemple est illustré figure 24.e et 24.f), d'un bandage, d'une bande adhésive 92 (dont un exemple est illustré figure 24.d). La figure 24.e montre un exemple d'électrode filaire formant un cercle. Ces accessoires ont aussi pour fonction de relier l'électrode au moyen 7, 7a et 7b de la connecter à la borne correspondante du générateur.

Dans un exemple préféré de l'invention, les électrodes non-invasives peuvent représenter un dispositif symétrique ou non symétrique, voire irrégulier. Dans le cas où les électrodes sont filaires 44a, 44b et non-invasives, ces électrodes peuvent suivre des axe non parallèles (dont un exemple est illustré figure 25).

Les électrodes non-invasives peuvent être pressées contre les tissus 50 afin de modifier leur géométrie pour augmenter le volume de tissus se trouvant entre les électrodes, comme exemplifié en figure 26 avec un support d'électrode en forme de pinces.

Les électrodes non-invasives peuvent aussi être pressées contre les tissus 50 pour diminuer la distance entre ces électrodes et d'autres électrodes reliées à une autre borne d'un générateur, comme exemplifié en en figure 27 avec une double électrode. Ceci permet notamment de se rapprocher d'une aiguille invasive introduite au sein d'une cavité.

### Description détaillée d'un mode de réalisation de l'invention.

La demanderesse présente ici un exemple préféré de l'invention décrit figure figures 12.a, 12.b et 12.c. Le dispositif est composé de 2 pièces principales:
- La "pièce d'injection" 23 rassemblant en particulier les électrodes, moyen d'injection et support
- l'habitacle 2 de la pièce d'injections, appelé "boîtier".

Le boîtier est composé de deux demi-cylindres 3, et l'ensemble est fixé à l'aide d'un anneau 22. La pièce d'injection 23 est constituée de 3 électrodes alignées, parallèles et de même profondeur, l'électrode centrale 11 se trouvant à distance égale entre les deux électrodes externes 100, 101 et permettant d'injecter le principe actif. Un orifice est prévu pour recevoir la seringue qui contiendra le principe actif qui sera injecté par l'électrode Première (11)

Les aiguilles sont assemblées à l'aide d'un ou deux disques de sertissage 41. Un film isolant électrique 15, composé d'une fine paroi en matière synthétique, est appliqué sur les électrodes sous le disque de sertissage 41.

Le boîtier sert d'habitacle pour la pièce d'injection 23. Il sert aussi de connexion électrique avec les bornes électriques de la pièce d'injection 2 et les fils électriques 7 reliés au générateur électrique 21 pour l'électrotransfert. Les deux électrodes 100,101 se trouvant à la périphérie de la pièce d'injection sont reliées à la même borne du générateur, l'électrode Première à l'autre borne. Le boîtier est composé de pièces ou plus, permettant, une fois assemblées, d'obtenir une prise en main aisée du dispositif. Le contact est réalisé par des formes métalliques 38 pressées contre l'électrode par un ressort ou une pièce élastique, ou aussi bien par des lamelles métalliques flexibles. Le boîtier sert aussi d'habitacle pour la seringue 1 contenant le principe actif 36 à injecter entre les deux électrodes externes de la pièce d'injection 23. Un système de butée 20 permet d'enfoncer le premier élément à une profondeur prédéfinie. La figure 13 montre le dispositif avec la pièce d'injection, composée de la seringue et de la pièce d'injection assemblée, positionnées dans son habitacle.

On définit le programme de séquence des impulsions, ou on sélectionne un programme préenregistré, qui définit notamment le nombre et la durée de chaque impulsion, l'intervalle entre les impulsions, la synchronisation des séquences et la tension à appliquer entre les électrodes.

On assemble la seringue et son produit actif avec la pièce d'électrode, et on les pose dans l'habitacle correspondant du boîtier, puis on assemble les deux demi cylindres, puis on verrouille le boîtier en enfilant le cylindre de verrouillage 22, figure 14, puis insère la butée 20 (figure 15). On branche les bornes électriques 7 au générateur d'impulsions électriques 21, on retire le capuchon protecteur des aiguilles 5. Le dispositif est assemblé (figure 15), et on peut alors le planter dans les tissus du sujet, selon les pratiques et gestes médicaux habituellement en pratique pour planter une aiguille munie d'une seringue. Le cylindre de verrouillage bloque le dispositif à une profondeur intermédiaire prédéfinie. On injecte le principe actif, on retire la butée, et on enfonce le boîtier jusqu'à la garde.

On peut maintenant déclencher les séries d'impulsion préprogrammées.

Puis, on déverrouille le boîtier et on jette les consommables: seringue 1, pièce d'injection 23 et son capuchon protecteur 5.

### Application industrielle de l'invention

Les applications industrielles de l'administration de nucléotides assistée par champs sont multiples. Le dispositif selon l'invention est particulièrement destiné à l'administration de médicaments, en particulier médicaments à base d'ADN, en médecine humaine et vétérinaire.

Les applications thérapeutiques chez l'homme ou l'animal concernent, en particulier, mais de façon bien évidemment non exhaustive, le traitement des tumeurs et la production de protéines sanguines.

La production de protéines dans le sang concerne le traitement de l'hémophilie, les troubles de la croissance, les myopathies, les maladies lysosomiales et métaboliques en général, l'insuffisance rénale chronique et la beta-thalassémie par la production endogène d'érythropoïétine. D'autres champs d'application concernent la néoangiogénèse, l'athérosclérose, en utilisant l'effet protecteur de cytokines telles que l'IL-10, la vaccination, l'utilisation d'oligonucléotides antisens, ou encore la prévention de la neuropathie périphérique induite par le cis-platine, par électrotransfert d'un plasmide codant pour une neurotrophine. Un accent particulier est mis actuellement sur l'utilisation dans la polyarthrite rhumatoïde articulaire ou dans des pathologies inflammatoires en général, en utilisant l'effet protecteur de l'IL-10, d'anti-TNF ou d'autres cytokines. L'utilisation de facteurs de croissance est aussi riche de potentiel dans des maladies neurodégénératives ou dégénératives de l'articulation (arthrose).

En outre, sont aussi concernées toutes les pathologies pouvant bénéficier de l'expression locale d'une protéine sécrétée, comme par exemple une protéine anti-inflammatoire secrétée dans l'articulation pour le traitement de l'arthrite, ou une protéine anti-angiogénique pour le traitement du cancer. De même, la production dans une articulation dans facteur de croissance peut être envisagée pour le traitement de l'arthrose. L'utilisation de protéines angiogéniques sécrétées localement pour le traitement de l'artérite périphérique est aussi envisageable.

Est aussi concerné la vaccination active et passive sur les êtres humains et les animaux, la fabrication de vaccins et la fabrication d'anti-corps, notamment dans la cadre des pathologies liées au bio-terrorisme. Il faut aussi mentionner les applications thérapeutiques pour lesquelles l'expression intracellulaire d'une protéine est nécessaire, comme de nombreuses maladies neuromusculaires(myopathie), les tumeurs, etc..

## Revendications

1. Dispositif pour améliorer la pénétration in vivo de molécules de principe actif dans les cellules des tissus d'un sujet humain ou animal, comprenant :
- un générateur (21) d'impulsions électriques comprenant des première et deuxième bornes et adapté pour émettre une séquence d'impulsions (131), la séquence d'impulsions ayant un intervalle de temps entre chaque Impulsion,
- au moins deux paires d'électrodes, chaque paire d'électrodes comprenant :
- une électrode d'un premier ensemble d'électrodes (11) composé d'au moins une électrode reliée électriquement à la première borne du générateur (21) d'impulsions, électriques,
- une électrode d'un deuxième ensemble d'électrodes (10) composé d'une pluralité d'électrodes reliées électriquement à la deuxième borne du générateur d'impulsions électriques, et
- un moyen (8) d'injecter le principe actif dans les tissus,
**caractérisé en ce que** le générateur (21) d'impulsions électriques est adapté pour émettre alternativement aux paires d'électrodes la séquence d'impulsions, deux séquences successives d'impulsions émises à deux paires différentes d'électrodes étant décalées d'une durée égale à l'intervalle de temps entre deux impulsions (131) de la séquence divisé par le nombre de paires d'électrodes.

2. Dispositif selon la revendication 1, **caractérisé en ce que** le premier ensemble d'électrodes est composé d'une seule électrode (11).

3. Dispositif selon la revendication 2, **caractérisé en ce que** l'électrode (11) est invasive.

4. Dispositif selon la revendication 3, **caractérisé en ce que** l'électrode (11) dispose d'un moyen d'injecter le principe actif.

5. Dispositif selon la revendication 4, **caractérisé en ce que** l'électrode (11) est une aiguille d'injection (8).

6. Dispositif selon la revendication 5, **caractérisé en ce qu'**un moyen d'injection du principe actif est constitué d'au moins une aiguille (8) solidaire et placée à proximité de l'électrode et de profondeur inférieure à la profondeur de l'électrode.

7. Dispositif selon la revendication 5, **caractérisé en ce que**:
- l'électrode est constituée en outre d'une enveloppe cathéter (70) percée d'orifices (78) de taille suffisante pour permettre un contact entre l'aiguille et les tissus au travers de ces orifices,
- L'aiguille invasive de l'électrode (71) traverse le cathéter par son axe et peut coulisser le long de l'axe du cathéter et pouvant être retirée du cathéter,
- Le cathéter et l'aiguille, une fois assemblés, forment une électrode invasive ces électrodes étant ci-après appelées "électrode recouverte partiellement d'un cathéter non conducteur" (75).

8. Dispositif selon l'une quelconque des revendications 4 à 6, **caractérisé en ce que** l'électrode (11) est constituée par :
- un cathéter (70), recouvert d'une surface conductrice électriquement (72) rellée à la borne correspondante du générateur d'impulsion, ci après nommé "électrode cathéter conductrice", sa surface étant composée d'un matériaux conservant approximativement la souplesse du cathéter, ce cathéter pouvant servir de moyen d'injection du principe actif,
- une aiguille invasive (71) traversant le cathéter par son axe et permettant de pénétrer les tissus et pouvant coulisser le long de l'axe du cathéter et pouvant être retirée du cathéter, l'aiguille pouvant aussi servir de moyen d'injection.

9. Dispositif selon l'une quelconque des revendications 3 à 8, **caractérisé en ce que** l'électrode Invasive est recouverte dans sa partie supérieure pénétrant dans les tissus, d'un isolant électrique (15).

10. Dispositif selon l'une quelconque des revendications 7 à 9, **caractérisé en ce qu'**au moins une électrode cathéter est aimantée (75).

11. Dispositif selon l'une quelconque des revendications 3 à 10, **caractérisé en ce que** le deuxième ensemble d'électrodes comporte au moins une électrode non-invasive (18) disposés à la surface des tissus recouvrant la zone contenant le principe actif.

12. Dispositif selon la revendication 11, **caractérisé en ce qu'**au moins une électrode non Invasive (18) dispose d'un orifice (43) permettant à l'électrode Invasive (11) de la traverser.

13. Dispositif selon la revendication 12, **caractérisé en ce qu'**elle comprend en outre un guide (49) permettant de diriger l'axe de l'électrode invasive (11) selon une direction prédéfinie.

14. Dispositif selon l'une quelconque des revendications 3 à 10, **caractérisé en ce que** le deuxième ensemble d'électrode comporte une pluralité d'électrodes invasives (10).

15. Dispositif selon la revendication 14, **caractérisé en ce que** l'électrode du deuxième ensemble est une électrode recouverte partiellement d'un cathéter non conducteur (75).

16. Dispositif selon la revendication 14, **caractérisé en ce que** le l'électrode du deuxième ensemble est constituée d'une électrode cathéter conductrice (75).

17. Dispositif selon l'une des revendications 14 ou 15, **caractérisé en ce que** l'électrode du premier ensemble est une électrode cathéter (75) et où les deux aiguilles sont parallèles. solidaires et sont reliées par un support.

18. Dispositif selon l'une quelconque des revendications 15 à 16, **caractérisé en ce que** les deux électrodes invasives (10,11) sont solidaires, assemblées à l'aide d'un support (41), approximativement parallèles et approximativement de même profondeur.

19. Dispositif selon l'une des revendications 14 à 18, **caractérisé en ce que** les électrodes invasives du deuxième ensemble (10) sont situées approximativement sur un cercle dont l'électrode (11) du premier ensemble forme approximativement au centre.

20. Dispositif selon l'une des revendications 14 à 19, **caractérisé en ce que** les électrodes invasives du deuxième ensemble bordent la zone où est injecté le principe actif (36).

21. Dispositif selon l'une quelconque des revendications 14 à 20, **caractérisé en ce que** deux électrodes invasives (100,101) du deuxième ensemble d'électrodes sont sensiblement alignées avec l'électrode du premier ensemble (11).

22. Dispositif selon l'une quelconque des revendications 14 à 21, **caractérisé en ce qu'**il comprend en outre un moyen (764,763) permettant d'orienter les électrodes selon un axe parallèle.

23. Dispositif selon l'une quelconque des revendications 19 à 21, **caractérisé en ce que** toutes les électrodes (10,11) sont solidaires, assemblées à l'aide d'un support (41), approximativement parallèles et approximativement de même profondeur.

24. Dispositif selon l'une quelconque des revendications 14 à 23, **caractérisé en ce que** le dispositif comprenne en outre au moins une électrode non-invasive reliée à l'une des bornes du générateur.

25. Dispositif selon l'une quelconque des revendications 4 à 24, **caractérisé en ce que** :
- Chaque électrode invasive peut disposer d'un boîtier (2) permettant une bonne prise en main de l'électrode et assurant la liaison électrique entre l'électrodes et sa borne du générateur (21),
- le boîtier (2) disposant d'un habitacle permettant de loger l'électrode et le moyen d'injection du principe actif et le réservoir (1) contenant le principe actif.

26. Dispositif selon l'une quelconque des revendications 4 à 24, **caractérisé en ce que** :
- une pluralité d'électrodes disposent du même boîtier (2) permettant une bonne prise en main du dispositif d'électrode et assurant la liaison électrique entre chaque électrode et sa borne du générateur (21) respective,
- le boîtier (2) dispose d'un habitacle permettant do loger les électrodes et le moyen d'injection du principe actif et le réservoir (1) contenant le principe actif.

27. Dispositif selon la revendication 22 ou 26, **caractérisé en ce que** le boîtier comprend en outre la moyen d'orienter les électrodes selon un axe parallèle.

28. Dispositif selon l'une des revendications 18 ou 23, **caractérisé en ce que** :
- les électrodes solidaires et leur support disposent d'un boîtier (2) permettant une bonne prise en main du dispositif d'électrode et assurant la liaison électrique entre chaque électrode et sa borne du générateur (21),
- le boîtier (2) dispose d'un habitacle permettant de loger les électrodes solidaires et leur support et le moyen d'injection du principe actif et le réservoir (1) contenant le principe actif.

29. Dispositif selon l'une des revendications 25 à 28, **caractérisé en ce que** le boîtier :
- dispose d'un moyen permettant d'enfoncer successivement l'électrode (11) dans les tissus à des profondeurs intermédiaires prédéfinies,
- et dispose d'un moyen permettant d'injecter le principe actif à chaque position d'arrêt.

30. Dispositif selon la revendication 29, **caractérisé en ce que** le moyen permettant d'enfoncer successivement les électrode dans les tissus à des profondeurs intermédiaires prédéfinies est [upsilon]b constitué d'au moins une butée (20).

31. Dispositif selon l'une quelconque des revendications 2 à 30, **caractérisé en ce qu'**une pluralité d'électrodes invasives reliées au générateur sont recouvertes dans leur partie supérieure pénétrant dans les tissus, d'un isolant électrique (15).

32. Dispositif selon l'une quelconque des revendications 2 à 31, **caractérisé en ce que** toutes les électrodes invasives reliées au générateur sont recouvertes dans leur partie supérieure pénétrant dans les tissus, d'un isolant électrique (15).

33. Dispositif selon la revendication 1, **caractérisé en ce que** les deux ensembles d'électrodes comportent un jeu d'électrodes non-invasives (18) disposées à la surface des tissus recouvrant la zone contenant le principe actif.

34. Dispositif selon la revendications 33, **caractérisé en ce que** les électrodes sont appliquées sur une même face des tissus (51) contenant le principe actif de façon à permettre la délivrance de champs se propageant sous la surface des tissus sur lesquels les électrodes sont appliquées.

35. Dispositif selon l'une quelconque des revendications 1, 11 à 13, 24, 33, 34, **caractérisé en ce que** la surface en contact avec les tissus d'au moins une électrode non-invasive (1 B) a une forme approximativement rectangulaire.

36. Dispositif selon l'une quelconque des revendications 1, 11 à 13, 24, 33, 34, **caractérisé en ce que** la surface en contact avec les tissus d'au moins une électrode non-invasive (18) a une forme ressemblant approximativement à un fer à cheval.

37. Dispositif selon l'une quelconque des revendications 1, 11 à 13, 24, 33 à 36, **caractérisé en ce que** la surface d'au moins une électrode non-invasive (18) est souple.

38. Dispositif selon l'une quelconque des revendications 1, 11 à 13, 24, 33, 34, **caractérisé en ce qu'**une électrode non-invasive (18) a la forme d'une pointe aplatie à son extrémité.

39. Dispositif selon l'une quelconque des revendications 1, 33 à 38, **caractérisé en ce que** les électrodes non-invasives font partie intégrante d'une gaine élastique (94).

40. Dispositif selon l'une quelconque des revendications 1, 11 à 13, 24, 33, 34, **caractérisé en ce qu'**au moins une électrode non-invasive (18) a une forme de fils.

41. Dispositif selon l'une quelconque des revendications 1, 33, 34, 40, **caractérise en ce que** le dispositif ne comporte que 2 électrodes non-invasives (18) en forme de fils, ces électrodes suivant un axe différent.

42. Dispositif selon l'une quelconque des revendications 1, 11 à 13, 24, 33, 34, 37, 39, **caractérisé en ce que** la surface en contact avec les tissus d'au moins une électrode non-invasive (18) a une forme irrégulière.

43. Dispositif selon l'une quelconque des revendications 1, 11 à 13, 24, 33 à 42, **caractérisé en ce que** les électrodes non-invasives (18) sont asymétriques.

44. Dispositif selon l'une quelconque des revendications 1, 11 à 13, 24, 33 à 43, **caractérisé en ce que** au moins une électrode non invasive est composée de plusieurs électrodes non invasive reliées électriquement, entre elles.

45. Dispositif selon l'une quelconque des revendications 1 à 44, comprenant des moyens pour mesurer la distance entre les électrodes, et des moyens pour calculer l'amplitude des signaux électriques en fonction de la distance entre les électrodes et de la nature des tissus.

46. Dispositif selon la revendication 45, **caractérisé en ce que** la position relative des électrodes est adaptée afin d'obtenir le champ électrique ou le courant électrique souhaités en fonction des tissus ciblés.

47. Dispositif selon la revendication 45, **caractérisé en ce que** le générateur (21) est programmé afin d'appliquer la tension permettant d'obtenir le champ électrique ou le courant électrique souhaité en fonction des tissus ciblés.

48. Dispositif selon la revendication 45, **caractérisé en ce que** le moyen pour mesurer la distance entre les électrodes permet do connaître en permanence la distance entre les extrémités des électrodes (74), et le générateur peut modifier dynamiquement la programmation des impulsions afin d'appliquer les paramètres permettant d'obtenir le champ électrique ou le courant électrique souhaités en fonction des tissus ciblés.

49. Dispositif selon l'une quelconque des revendications 1 à 48, **caractérisé en ce que** la durée (131) entre chaque impulsions électrique émise par le générateur (21) vers une paire d'électrode est comprise entre 1 ms et 50 ms, afin d'améliorer l'innocuité et do diminuer l'intensité des contractions musculaires.

50. Dispositif selon l'une quelconque des revendication 1 à 49, **caractérisé en ce que** les impulsions électriques émises par le générateur (21) sont unipolaires et ont une forme carrée.

51. Dispositif selon l'une quelconque des revendications 1 à 50, **caractérisé en ce que**:
- le rapport entre la différence de potentiel entre chaque électrode et leur distance est compris entre 10 Volt/cm et 750 Volt/cm,
- la durée des impulsions électriques (132) est comprise entre 1 et 250 ms,
- la durée entre les impulsions électriques (131) est comprise entre 1 et 1500 ms,
- le nombre d'impulsions de chaque séquence d'impulsions (133) est compris entre 1 et 1000.

52. Dispositif selon l'une quelconque des revendications 1 à 51, **caractérisé en ce que** le rapport Volt/cm appliqué entre les électrodes (10, 11, 18, 76) prend une valeur comprise entre 1,05 et 1,50 fois la valeur optimum des champs pour les tissus ciblés afin d'augmenter le volume de tissus contenant la principe actif traversé par des champs à nombre de Volt/cm optimum.

## Patentansprüche

1. Vorrichtung zur Verbesserung des Eindringens von Wirkstoff-Molekülen in die Zellen menschlicher oder tierischer Gewebe in vivo, aufweisend:
- einen Generator (21) von elektrischen Impulsen, der eine erste und zweite Anschlußklemme aufweist und eingerichtet ist, um eine Folge von Impulsen (131) auszusenden, wobei die Impulsfolge ein Zeitintervall zwischen jedem Impuls hat,
- mindestens zwei Elektrodenpaare, wobei jedes Elektrodenpaar aufweist:
- eine Elektrode einer ersten Elektrodengruppe (11), die mindestens aus einer Elektrode besteht, die mit der ersten Anschlußklemme des Generators (21) von elektrischen Impulsen elektrisch verbunden ist,
- eine Elektrode einer zweiten Elektrodengruppe (10), die aus mehreren Elektroden besteht, die mit der zweiten Anschlußklemme des Generators von elektrischen Impulsen elektrisch verbunden sind, und
- eine Einrichtung (8) zum Injizieren des Wirkstoffs in die Gewebe,
**dadurch gekennzeichnet, daß** der Generator (21) von elektrischen Impulsen eingerichtet ist, um die Impulsfolge abwechselnd zu den Elektrodenpaaren auszusenden, wobei zwei aufeinanderfolgende Impulsfolgen, die zu zwei unterschiedlichen Paaren ausgesandt werden, um eine Dauer zueinander verschoben sind, die gleich dem Zeitintervall zwischen zwei Impulsen (131) der Folge geteilt durch die Anzahl von Elektrodenpaaren ist.

2. Vorrichtung nach Anspruch 1, **dadurch gekennzeichnet, daß** die ersten Elektrodengruppe aus einer einzigen Elektrode (11) besteht.

3. Vorrichtung nach Anspruch 2, **dadurch gekennzeichnet, daß** die Elektrode (11) invasiv ist.

4. Vorrichtung nach Anspruch 3, **dadurch gekennzeichnet, daß** die Elektrode über eine Einrichtung zum Injizieren des Wirkstoffs verfügt.

5. Vorrichtung nach Anspruch 4, **dadurch gekennzeichnet, daß** die Elektrode (11) eine Injektionsnadel (8) ist.

6. Vorrichtung nach Anspruch 5, **dadurch gekennzeichnet, daß** eine Einrichtung zum Injizieren des Wirkstoffs aus mindestens einer Nadel (8) besteht, die mit der Elektrode fest verbunden ist und in der Nähe der Elektrode und in einer niedrigeren Tiefe als die Tiefe der Elektrode angeordnet ist.

7. Vorrichtung nach Anspruch 5, **dadurch gekennzeichnet, daß**:
- die Elektrode außerdem aus einer Katheterhülle (70) besteht, die mit Öffnungen (78) einer Größe durchbohrt ist, die ausreichend ist, um über diese Öffnungen einen Kontakt zwischen der Nadel und den Geweben zu erlauben,
- die invasive Nadel der Elektrode (71) durch den Katheter über dessen Achse hindurchgeht und entlang der Achse des Katheters gleiten kann und von dem Katheter zurückgezogen werden kann,
- der Katheter und die Nadel, nachdem sie zusammengebaut worden sind, eine invasive Elektrode bilden, wobei diese Elektroden nachstehend als "eine teilweise mit einem nichtleitenden Katheter bedeckte Elektrode" (75) bezeichnet werden.

8. Vorrichtung nach einem der Ansprüche 4 bis 6, **dadurch gekennzeichnet, daß** die Elektrode (11) besteht aus:
- einem Katheter (70), der mit einer elektrisch leitenden Oberfläche (72) bedeckt ist, die mit der entsprechenden Anschlußklemme des Impulsgenerators verbunden ist, hier nachstehend "leitende Katheterelektrode" genannt, wobei seine Oberfläche aus einem Material besteht, das die Flexibilität des Katheters im wesentlichen bewahrt, wobei dieser Katheter als Einrichtung zum Injizieren des Wirkstoffs dienen kann,
- einer invasiven Nadel (71), die durch den Katheter über dessen Achse hindurchgeht und ein Eindringen in die Gewebe erlaubt und entlang der Achse des Katheters gleiten kann und von dem Katheter zurückgezogen werden kann, wobei die Nadel auch als Injektionseinrichtung dienen kann.

9. Vorrichtung nach einem der Ansprüche 3 bis 8, **dadurch gekennzeichnet, daß** die invasive Elektrode in ihrem in die Gewebe eindringenden oberen Teil mit einer elektrischen Isolierung (15) bedeckt ist.

10. Vorrichtung nach einem der Ansprüche 7 bis 9, **dadurch gekennzeichnet, daß** mindestens eine Katheterelektrode magnetisch (75) ist.

11. Vorrichtung nach einem der Ansprüche 3 bis 10, **dadurch gekennzeichnet, daß** die zweite Elektrodengruppe mindestens eine nichtinvasive Elektrode (18) aufweist, die an der Oberfläche der Gewebe angeordnet wird, wobei die Zone, die den Wirkstoff enthält, bedeckt wird.

12. Vorrichtung nach Anspruch 11, **dadurch gekennzeichnet, daß** mindestens eine nichtinvasive Elektrode (18) mit einer Öffnung (43) versehen ist, die einen Durchgang der invasiven Elektrode (11) durch die nichtinvasive Elektrode erlaubt.

13. Vorrichtung nach Anspruch 12, **dadurch gekennzeichnet, daß** sie außerdem eine Führung (49) aufweist, die erlaubt, die Achse der invasiven Elektrode (11) in eine vorgegebene Richtung zu lenken.

14. Vorrichtung nach einem der Ansprüche 3 bis 10, **dadurch gekennzeichnet, daß** die zweite Elektrodengruppe mehrere invasive Elektroden (10) aufweist.

15. Vorrichtung nach Anspruch 14, **dadurch gekennzeichnet, daß** die Elektrode der zweiten Gruppe eine Elektrode ist, die teilweise mit einem nichtleitenden Katheter (75) bedeckt ist.

16. Vorrichtung nach Anspruch 14, **dadurch gekennzeichnet, daß** die Elektrode der zweiten Gruppe aus einer leitenden Katheterelektrode (75) besteht.

17. Vorrichtung nach einem der Ansprüche 14 oder 15, **dadurch gekennzeichnet, daß** die Elektrode der ersten Gruppe eine Katheterelektrode (75) ist und wobei die zwei Nadeln parallel sind und über einen Träger fest miteinander verbunden sind.

18. Vorrichtung nach einem der Ansprüche 15 bis 16, **dadurch gekennzeichnet, daß** die zwei invasiven Elektroden (10,11) fest miteinander verbunden sind, mit Hilfe eines Trägers (41) zusammengebaut sind, ungefähr parallel und ungefähr von einer gleichen Tiefe sind.

19. Vorrichtung nach einem der Ansprüche 14 bis 18, **dadurch gekennzeichnet, daß** die invasiven Elektroden der zweiten Gruppe (10) ungefähr auf einem Kreis angeordnet sind, wobei die Elektrode (11) der ersten Gruppe ungefähr das Zentrum bildet.

20. Vorrichtung nach einem der Ansprüche 14 bis 19, **dadurch gekennzeichnet, daß** die invasiven Elektroden der zweiten Gruppe die Zone begrenzen, in welche der Wirkstoff (36) injiziert wird.

21. Vorrichtung nach einem der Ansprüche 14 bis 20, **dadurch gekennzeichnet, daß** zwei invasive Elektroden (100,101) der zweiten Elektrodengruppe im wesentlichen mit der Elektrode der ersten Gruppe (11) ausgerichtet sind.

22. Vorrichtung nach einem der Ansprüche 14 bis 21, **dadurch gekennzeichnet, daß** sie außerdem eine Einrichtung (764, 763) aufweist, die erlaubt, die Elektroden gemäß einer parallelen Achse auszurichten.

23. Vorrichtung nach einem der Ansprüche 19 bis 21, **dadurch gekennzeichnet, daß** alle Elektroden (10,11) miteinander verbunden sind, mit Hilfe eines Trägers (41) zusammengebaut sind, ungefähr parallel und ungefähr von gleicher Tiefe sind.

24. Vorrichtung nach einem der Ansprüche 14 bis 23, **dadurch gekennzeichnet, daß** die Vorrichtung außerdem mindestens eine nichtinvasive Elektrode aufweist, die mit einer der Anschlußklemmen des Generators verbunden ist.

25. Vorrichtung nach einem der Ansprüche 4 bis 24, **dadurch gekennzeichnet, daß**:
- jede invasive Elektrode mit einem Gehäuse (2) versehen sein kann, das ein gutes manuelles Greifen der Elektrode erlaubt und die elektrische Verbindung zwischen der Elektrode und der ihr zugehörigen Anschlußklemme des Generators (21) sicherstellt,
- das Gehäuse (2) mit einer Aufnahme versehen ist, die erlaubt, die Elektrode und die Wirkstoff-Injektionseinrichtung und den Behälter (1), der den Wirkstoff enthält, unterzubringen.

26. Vorrichtung nach einem der Ansprüche 4 bis 24, **dadurch gekennzeichnet, daß**:
- mehrere Elektroden das gleiche Gehäuse (2) aufweisen, das ein gutes manuelles Greifen der Elektrodenvorrichtung erlaubt und die elektrische Verbindung zwischen jeder Elektrode und der jeweiligen Anschlußklemme des Generators (21) sicherstellt,
- das Gehäuse (2) mit einer Aufnahme versehen ist, die erlaubt, die Elektroden und die Wirkstoff-Injektionseinrichtung und den Behälter (1), der den Wirkstoff enthält, unterzubringen.

27. Vorrichtung nach Anspruch 22 oder 26, **dadurch gekennzeichnet, daß** das Gehäuse außerdem die Einrichtung zum Ausrichten der Elektroden gemäß einer parallelen Achse aufweist.

28. Vorrichtung nach einem der Ansprüche 18 oder 23, **dadurch gekennzeichnet, daß**:
- die fest miteinander verbundenen Elektroden und ihr Träger mit einem Gehäuse (2) versehen sind, das ein gutes manuelles Greifen der Elektrodenvorrichtung erlaubt und die elektrische Verbindung zwischen jeder Elektrode und der ihr zugehörigen Anschlußklemme des Generators (21) sicherstellt,
- das Gehäuse (2) mit einer Aufnahme versehen ist, die erlaubt, die miteinander verbundenen Elektroden und ihren Träger und die Wirkstoff-Injektionseinrichtung und den Behälter (1), der den Wirkstoff enthält, unterzubringen.

29. Vorrichtung nach einem der Ansprüche 25 bis 28, **dadurch gekennzeichnet, daß** das Gehäuse
- über eine Einrichtung verfügt, die erlaubt, die Elektrode (11) nacheinander in vorgegebene Zwischentiefen in die Gewebe hineinzutreiben,
- und über eine Einrichtung verfügt, die erlaubt, an jeder Halteposition den Wirkstoff zu injizieren.

30. Vorrichtung nach Anspruch 29, **dadurch gekennzeichnet, daß** die Einrichtung, die erlaubt, die Elektrode nacheinander in vorgegebene Zwischentiefen in die Gewebe hineinzutreiben, [Upsilon]b ist, das aus mindestens einem Anschlag (20) besteht.

31. Vorrichtung nach einem der Ansprüche 2 bis 30, **dadurch gekennzeichnet, daß** mehrere mit dem Generator verbundene invasive Elektroden in ihrem in die Gewebe eindringenden oberen Teil mit einer elektrischen Isolierung (15) bedeckt sind.

32. Vorrichtung nach einem der Ansprüche 2 bis 31, **dadurch gekennzeichnet, daß** alle mit dem Generator verbundene invasive Elektroden in ihrem in die Gewebe eindringenden oberen Teil mit einer elektrischen Isolierung (15) bedeckt sind.

33. Vorrichtung nach Anspruch 1, **dadurch gekennzeichnet, daß** die zwei Elektrodengruppen einen Satz von nichtinvasiven Elektroden (18) aufweisen, die an der Oberfläche der Gewebe angeordnet werden, wobei die Zone, die den Wirkstoff enthält, bedeckt wird.

34. Vorrichtung nach Anspruch 33, **dadurch gekennzeichnet, daß** die Elektroden auf einer gleichen Fläche der Gewebe (51) angelegt werden, die den Wirkstoff enthalten, um so die Abgabe von Feldern zu erlauben, die sich unterhalb der Oberfläche der Gewebe, auf welchen die Elektroden angelegt sind, ausbreiten.

35. Vorrichtung nach einem der Ansprüche 1, 11 bis 13, 24, 33, 34, **dadurch gekennzeichnet, daß** die mit den Geweben in Kontakt stehende Oberfläche mindestens einer nichtinvasiven Elektrode (18) eine ungefähr rechteckige Form hat.

36. Vorrichtung nach einem der Ansprüche 1, 11 bis 13, 24, 33, 34, **dadurch gekennzeichnet, daß** die mit den Geweben in Kontakt stehende Oberfläche mindestens einer nichtinvasiven Elektrode (18) eine Form hat, die ungefähr einem Hufeisen ähnlich ist.

37. Vorrichtung nach einem der Ansprüche 1, 11 bis 13, 24, 33 bis 36, **dadurch gekennzeichnet, daß** die Oberfläche mindestens einer nichtinvasiven Elektrode (18) flexibel ist.

38. Vorrichtung nach einem der Ansprüche 1, 11 bis 13, 24, 33, 34, **dadurch gekennzeichnet, daß** eine nichtinvasive Elektrode (18) die Form einer an ihrem Ende abgeplatteten Spitze hat.

39. Vorrichtung nach einem der Ansprüche 1, 33 bis 38, **dadurch gekennzeichnet, daß** die nichtinvasiven Elektroden einen integralen Bestandteil einer elastischen Ummantelung (94) bilden.

40. Vorrichtung nach einem der Ansprüche 1, 11 bis 13, 24, 33, 34, **dadurch gekennzeichnet, daß** mindestens eine nichtinvasive Elektrode (18) die Form von Drähten hat.

41. Vorrichtung nach einem der Ansprüche 1, 33, 34, 40, **dadurch gekennzeichnet, daß** die Vorrichtung nur zwei nichtinvasive Elektroden (18) in Form von Drähten aufweist, wobei diese Elektroden eine unterschiedliche Achse haben.

42. Vorrichtung nach einem der Ansprüche 1, 11 bis 13, 24, 33, 34, 37, 39, **dadurch gekennzeichnet, daß** die mit den Geweben in Kontakt stehende Oberfläche mindestens einer nichtinvasiven Elektrode (18) eine unregelmäßige Form hat.

43. Vorrichtung nach einem der Ansprüche 1, 11 bis 13, 24, 33 bis 42, **dadurch gekennzeichnet, daß** die nichtinvasiven Elektroden (18) asymmetrisch sind.

44. Vorrichtung nach einem der Ansprüche 1, 11 bis 13, 24, 33 bis 43, **dadurch gekennzeichnet, daß** mindestens eine nichtinvasive Elektrode aus mehreren elektrisch miteinander verbundenen nichtinvasiven Elektroden gebildet ist.

45. Vorrichtung nach einem der Ansprüche 1 bis 44, mit Einrichtungen zum Messen des Abstands zwischen den Elektroden und Einrichtungen zum Berechnen der Amplitude von elektrischen Signalen als Funktion des Abstands zwischen den Elektroden und der Art der Gewebe.

46. Vorrichtung nach Anspruch 45, **dadurch gekennzeichnet, daß** die relative Position der Elektroden angepaßt wird, um das gewünschte elektrische Feld oder den gewünschten elektrischen Strom in Abhängigkeit von den Zielgeweben bereitzustellen.

47. Vorrichtung nach Anspruch 45, **dadurch gekennzeichnet, daß** der Generator (21) programmiert wird, um die Spannung anzulegen, die erlaubt, das gewünschte elektrische Feld oder den gewünschten elektrischen Strom in Abhängigkeit von den Zielgeweben bereitzustellen.

48. Vorrichtung nach Anspruch 45, **dadurch gekennzeichnet, daß** die Einrichtung zum Messen des Abstands zwischen den Elektroden erlaubt, den Abstand zwischen den Enden der Elektroden (74) kontinuierlich zu kennen, und der Generator dynamisch die Programmierung der Impulse modifizieren kann, um die Parameter anzuwenden, die erlauben, das gewünschte elektrische Feld oder den gewünschten elektrischen Strom in Abhängigkeit von den Zielgeweben bereitzustellen.

49. Vorrichtung nach einem der Ansprüche 1 bis 48, **dadurch gekennzeichnet, daß** die Dauer (131) zwischen jedem von dem Generator (12) zu einem Elektrodenpaar ausgegebenen elektrischen Impuls zwischen 1ms und 50ms beträgt, um die Unschädlichkeit zu verbessern und die Intensität von Muskelkontraktionen zu verringern.

50. Vorrichtung nach einem der Ansprüche 1 bis 49, **dadurch gekennzeichnet, daß** die von dem Generator (21) ausgesandten elektrischen Impulse einpolig sind und eine Rechteckform haben.

51. Vorrichtung nach einem der Ansprüche 1 bis 50, **dadurch gekennzeichnet, daß**:
- das Verhältnis zwischen der Potentialdifferenz zwischen jeder Elektrode und ihrem Abstand zwischen 10 Volt/cm und 750 Volt/cm beträgt,
- die Dauer der elektrischen Impulse (132) zwischen 1 und 250ms beträgt,
- die Dauer zwischen den elektrischen Impulsen (131) zwischen 1 und 1500ms beträgt,
- die Anzahl der Impulse jeder Impulsfolge (133) zwischen 1 und 1000 beträgt.

52. Vorrichtung nach einem Ansprüche 1 bis 51, **dadurch gekennzeichnet, daß** das zwischen den Elektroden (10, 11, 18, 75) angelegte Volt/cm-Verhältnis einen Wert annimmt, der zwischen dem 1,05- und 1,50-fachen des optimalen Werts der Felder für die Zielgewebe liegt, um das den Wirkstoff enthaltende Gewebevolumen zu vergrößern, durch welches Felder einer optimalen Anzahl von Volt/cm hindurchgehen.

## Claims

1. Device for improving the in vivo penetration of molecules of active ingredient into the cells of the tissues of a human or animal subject, **characterized in that** it comprises:
- a generator of electric pulses (21) comprising first and second terminals and adapted to emit a sequence of pulses (131), the sequence of pulses (131) having a time interval between each pulse,
- at least two pairs of electrodes, wherein each pair of electrodes comprises:
- an electrode of a first group of electrodes (11) composed of at least one electrode electrically connected to the first terminal of the generator (21) of electric pulses,
- an electrode of a second group of electrodes (10) composed of a plurality of electrodes electrically connected to the second terminal of the generator of electric pulses, and
- a means (8) of injecting the active ingredient into the tissues,
**characterized in that** the generator (21) of electric pulses is adapted to emit alternatively to the pairs of electrodes the sequence of pulses, two successive sequences of pulses emitted to two different pairs of electrodes being offset of a duration equal to the time interval between two pulses (131) of the sequence divided by the number of pairs of electrodes.

2. Device according to claim 1, **characterized in that** the first group of electrodes is composed of a single electrode (11).

3. Device according to claim 2, **characterized in that** the electrode (11) is invasive.

4. Device according to claim 3, **characterized in that** the electrode (11) has a means of injecting the active ingredient.

5. Device according to claim 4, **characterized in that** the electrode (11) is an injection needle (8).

6. Device according to claim 5, **characterized in that** a means of injecting the active ingredient is constituted by at least one integral needle (8) and placed close to the electrode and at a depth less than the depth of the electrode.

7. Device according to claim 5, **characterized in that**:
- the electrode is further constituted by a catheter envelope (70) pierced by orifices (78) of sufficient size to permit a contact between the needle and the tissues through these orifices,
- the invasive needle of the electrode (71) passes through the catheter along its axis and can slide along the axis of the catheter and can be withdrawn from the catheter,
- the catheter and the needle, once assembled, form an invasive electrode, these electrodes being hereinafter called "electrode partly covered by a non-conductive catheter" (75)

8. Device according to any one of claims 4 to 6, **characterized in that** the electrode (11) is constituted by:
- a catheter (70), covered by an electrically conductive surface (72) connected to the corresponding terminal of the pulse generator, hereinafter called "conductive catheter electrode", its surface being composed of a material approximately retaining the flexibility of the catheter, which catheter can serve as means of injecting the active ingredient
- an invasive needle (71) passing through the catheter along its axis and allowing penetration of the tissues and able to slide along the axis of the catheter and able to be withdrawn from the catheter, which needle can also serve as injection means.

9. Device according to any one of claims 3 to 8, **characterized in that** the invasive electrode is covered, in its upper part penetrating into the tissues, by an electric insulating material (15).

10. Device according to any one of claims 7 to 9, **characterized in that** at least one catheter electrode is magnetized (75).

11. Device according to any one of claims 3 to 10, **characterized in that** the second group of electrodes comprises at least one non-invasive electrode (18) arranged on the surface of the tissues covering the zone containing the active ingredient.

12. Device according to claim 11, **characterized in that** at least one non-invasive electrode (18) has an orifice (43) allowing the invasive electrode (11) to pass through it.

13. Device according to claim 12, **characterized in that** it also comprises a guide (49) allowing the axis of the invasive electrode (11) to be directed in a predefined direction.

14. Device according to any one of claims 3 to 10, **characterized in that** the second group of electrodes comprises a plurality of invasive electrodes (10).

15. Device according to claim 14, **characterized in that** the electrode of the second group is an electrode partly covered by a non-conductive catheter (75).

16. Device according to claim 14, **characterized in that** the electrode of the second group is constituted by a conductive catheter electrode (75).

17. Device according to one of claims 14 or 15, **characterized in that** the electrode of the first group is a catheter electrode (75) and where the two needles are parallel, integral and connected by a support.

18. Device according to any one of claims 15 to 16, **characterized in that** the two invasive electrodes (10,11) are integral, assembled using a support (41), approximately parallel and approximately of the same depth.

19. Device according to any one of claims 14 to 18, **characterized in that** the invasive electrodes of the second group (10) are situated approximately on a circle of which the electrode (11) of the first group approximately forms the centre.

20. Device according to one of claims 14 to 19, **characterized in that** the invasive electrodes of the second group border the zone where the active ingredient is injected (36).

21. Device according to any one of claims 14 to 20, **characterized in that** two invasive electrodes (100, 101) of the second group of electrodes are substantially aligned with the electrode of the first group (11).

22. Device according to any one of claims 14 to 21, **characterized in that** it also comprises a means (764, 763) allowing orientation of the electrodes along a parallel axis.

23. Device according to any one of claims 19 to 21, **characterized in that** all the electrodes (10, 11) are integral, assembled using a support (41), approximately parallel and approximately of the same depth.

24. Device according to any one of claims 14 to 23, **characterized in that** the device also comprises at least one non-invasive electrode connected to one of the terminals of the generator.

25. Device according to any one of claims 4 to 24 **characterized in that**:
- each invasive electrode can have a casing (2) allowing a good grip on the electrode and providing the electric connection between the electrodes and its terminal of the generator (21).
- the casing (2) having a housing allowing the electrodes and the means of injecting the active ingredient and the reservoir (1) containing the active ingredient to be accommodated.

26. Device according to any one of claims 4 to 24, **characterized in that**:
- a plurality of electrodes have a single casing (2) allowing a good grip on the electrode device and providing the electric connection between each electrode and its terminal of the respective generator (21),
- the casing (2) has a housing allowing the electrodes and the means of injecting the active ingredient and the reservoir (1) containing the active ingredient to be accommodated.

27. Device according to claim 22 or 26, **characterized in that** the casing also comprises the means of orienting the electrodes along a parallel axis.

28. Device according to one of claims 18 or 23, **characterized in that**:
- the integral electrodes and their support have a casing (2) allowing a good grip on the electrode device and providing the electric connection between each electrode and its terminal of the generator (21),
- the casing (2) has a housing allowing the integral electrodes and their support and the means of injecting the active ingredient and the reservoir (1) containing the active ingredient to be accommodated.

29. Device according to one of claims 25 to 28, **characterized in that** the casing:
- has a means allowing the electrode (11) to be successively pushed into the tissues to predefined intermediate depths,
- and has a means allowing the injection of the active ingredient at each stop position.

30. Device according to claim 29, **characterized in that** the means allowing the electrode (11) to be successively pushed into the tissues to predefined intermediate depths is constituted by at least one stop (20).

31. Device according to any one of claims 2 to 30, **characterized in that** a plurality of invasive electrodes connected to the generator are covered, in their upper part penetrating into the tissues, by an electric insulating material (15).

32. Device according to any one of claims 2 to 31, **characterized in that** all the invasive electrodes connected to the generator are covered, in their upper part penetrating into the tissues, by an electric insulating material (15).

33. Device according to claim 1, **characterized in that** the two groups of electrodes comprise a set of non-invasive electrodes (18) arranged on the surface of the tissues covering the zone containing the active ingredient.

34. Device according to the claim 33, **characterized in that** the electrodes are applied to a single face of the tissues (51) containing the active ingredient so as permit the delivery of fields spreading under the surface of the tissues to which the electrodes are applied.

35. Device according to any one of claims 1, 11 to 13, 24, 33, 34, **characterized in that** the surface in contact with the tissues of at least one non-invasive electrode (18) has an approximately rectangular shape.

36. Device according to any one of claims 1, 11 to 13, 24, 33, 34, **characterized in that** the surface in contact with the tissues of at least one non-invasive electrode (18) has a shape approximately resembling a horseshoe.

37. Device according to any one of claims 1, 11 to 13, 24, 33 to 36, **characterized in that** the surface of at least one non-invasive electrode (18) is flexible.

38. Device according to any one of claims 1, 11 to 13, 24, 33, 34, **characterized in that** a non-invasive electrode (18) has the shape of a tip flattened at its end.

39. Device according to any one of claims 1, 33 to 38, **characterized in that** the non-invasive electrodes form an integral part of an elastic sheath (94).

40. Device according to any one of claims 1, 11 to 13, 24, 33,34, **characterized in that** at least one non-invasive electrode (18) is wire-shaped.

41. Device according to any one of claims 1, 33, 34, 40, **characterized in that** the device comprises only 2 wire-shaped non-invasive electrodes (18), these electrodes following a different axis.

42. Device according to any one of claims 1, 11 to 13, 24, 33, 34, 37, 39, **characterized in that** the surface in contact with the tissues of at least one non-invasive electrode (18) has an irregular shape.

43. Device according to any one of claims 1, 11 to 13, 24, 33 to 42, **characterized in that** the non-invasive electrodes (18) are asymmetrical.

44. Device according to any one of claims 1, 11 to 13, 24, 33 to 43, **characterized in that** at least one non-invasive electrode is composed of several non-invasive electrodes electrically connected to one another.

45. Device according to any one of claims 1 to 44, comprising a means is used to ascertain the distance between the electrodes and a means to calculate the amplitude of the electric signals as a function of the distance between the electrodes and the nature of the tissue.

46. Device according to claim 45, **characterized in that** the relative position of the electrodes is adapted in order to obtain the electric field or electric current desired according to the targeted tissues.

47. Device according to claim 45, **characterized in that** the generator (21) is programmed in order to apply the voltage allowing the desired electric field or electric current to be obtained according to the targeted tissues.

48. Device according to claim 45, **characterized in that** the means of ascertaining the distance between the electrodes enables to continuously ascertain the distance between the ends of the electrodes (74), and the generator can dynamically modify the programming of the pulses in order to apply the parameters allowing the desired electric field or electric current to be obtained according to the targeted tissues.

49. Device according to any one of claims 1 to 48, **characterized in that** the duration (131) between each electric pulse emitted by the generator (21) towards a pair of electrodes is comprised between 1 ms and 50 ms, in order to reduce the harmfulness and reduce the intensity of the muscular contractions.

50. Device according to any one of claims 1 to 49, **characterized in that** the electric pulses emitted by the generator (21) are unipolar and have a square shape.

51. Device according to any one of claims 1 to 50, **characterized in that**:
- the ratio between the potential difference between each electrode and their distance is comprised between 10 volt/cm and 750 volt/cm,
- the duration of the electric pulses (132) is comprised between 1 and 250 ms,
- the duration between the electric pulses (131) is comprised between 1 and 1500 ms,
- the number of pulses of each sequence of pulses (133) is comprised between 1 and 1000.

52. Device according to any one of claims 1 to 51, **characterized in that** the volt/cm ratio applied between the electrodes (10, 11, 18, 75) assumes a value comprised between 1.05 and 1.50 times the optimum value of the fields for the targeted tissues in order to increase the volume of tissues containing the active ingredient passed through by fields at an optimum number of volt/cm.
